# FASCICULE DE BREVET EUROPEEN

(11) **EP 0 979 244 B1**
(45) Date de publication et mention de la délivrance du brevet: **02.03.2005**
(21) Numéro de dépôt: 98922872.1
(22) Date de dépôt: 27.04.1998
(51) Int. Cl.: C07K 7/64, A61K 38/13

(54) **DERIVES DE CYCLOSPORINE, LEUR PREPARATION ET LES COMPOSITIONS PHARMACEUTIQUES QUI LES CONTIENNENT**
CYCLOSPORINDERIVATE, DEREN HERSTELLUNG UND PHARMAZEUTISCHE ZUBEREITUNGEN
NOVEL CYCLOSPORIN DERIVATIVES, PREPARATION AND PHARMACEUTICAL COMPOSITIONS CONTAINING SAME

(30) Priorité: 30.04.1997 FR 9705351
(43) Date de publication de la demande: 16.02.2000
(73) Titulaire: Aventis Pharma S.A., 92160 Antony (FR)
(72) Inventeur: EVERS, Michel, F-94510 La Queue en Brie (FR); MIGNANI, Serge, F-92290 Châtenay Malabry (FR); CARRY, Jean-Christophe, F-92190 Meudon (FR); FILOCHE, Bruno, F-94000 Créteil (FR); BASHIARDES, Georges, F-86000 Poitiers (FR); BENSOUSSAN, Claude, F-94550 Chevilly Larue (FR); GUEGUEN, Jean-Christophe, F-92290 Châtenay Malabry (FR); BARRIERE, Jean-Claude, F-91400 Bures sur Yvette (FR)
(86) Numéro de dépôt international: PCT/FR1998/000838
(87) Numéro de publication internationale: WO 1998/049193

(56) Documents cités:
- EP-A- 0 194 972
- EP-A- 0 484 281
- WO-A-97/04005
- GB-A- 2 205 317
- US-A- 4 814 323
- BILLICH A ET AL: "MODE OF ACTION OF SDZ NIM 811, A NONIMMUNOSUPPRESSIVE CYCLOSPORIN A ANALOG WITH ACTIVITY AGAINST HUMAN IMMUNODEFICIENCY VIRUS (HIV) TYPE 1: INTERFERENCE WITH HIV PROTEIN-CYCLOPHILIN A INTERACTIONS" JOURNAL OF VIROLOGY, vol. 69, no. 4, avril 1995, pages 2451-2461, XP002022423

## Description

La présente invention concerne de nouveaux dérivés de cyclosporine de formule générale : 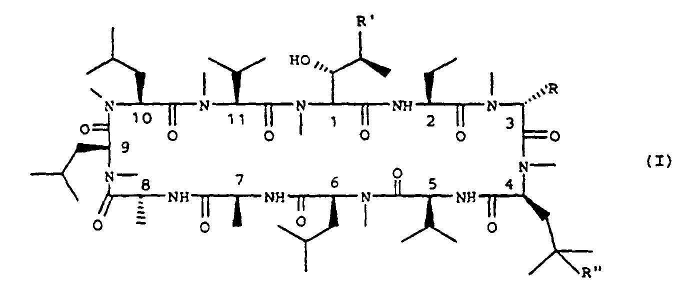 leurs sels, leur préparation et les compositions pharmaceutiques qui les contiennent.

Les dérivés de formule générale (I) sont utiles pour le traitement et/ou la prophylaxie des infections à rétrovirus, et plus particulièrement du SIDA (syndrome d'immuno-déficience acquise) et de syndromes associés [ARC (AIDS related complex)].

Des dérivés de cyclosporine respectivement modifiés en position -1 ou -3 ont été précédemment décrits comme agents immunosuppresseurs, dans la demande de brevet britannique 2 205 317 et dans le brevet européen EP 194 972.

Des dérivés de cyclosporine modifiés en position -1 par des chaînes très courtes ont été décrits dans par S. Bartz dans Proc. Natl. Acad. Sci. USA 92, 5381 (1995) comme agents utiles dans le traitement du SIDA, cependant des chaînes plus longues conduisaient à la disparition de l'activité.

Des dérivés de cyclosporine diversement modifiés et notamment le dérivé [4'-hydroxy-MeLeu]⁴ cyclosporine, ont été précédemment décrits dans le brevet européen EP 484281 et dans Eur. J. Immunol., 17, 1359 (1987). Ces dérivés sont utiles pour le traitement du SIDA.

Il a été trouvé maintenant que les dérivés de la cyclosporine de formule générale (I) dans laquelle :
R est un atome d'hydrogène ou un radical de structure :

   ―S―Alk-R° (Ia)

   pour lequel
   - Alk-R° représente un radical méthyle, ou bien
   - Alk représente un radical alcoylène contenant 2 à 6 atomes de carbone en chaîne droite ou ramifiée ou cycloalcoylène contenant 3 à 6 atomes de carbone et
   - R° représente
      · soit un atome d'hydrogène, un radical hydroxy, carboxy ou alcoyloxycarbonyle,
      · soit un radical -NR₁R₂ pour lequel R₁ et R₂ identiques ou différents représentent des atomes d'hydrogène, ou des radicaux alcoyle, alcényle (2 à 4C), cycloalcoyle (3 à 6C), phényle éventuellement substitué (par un atome d'halogène, alcoyloxy, alcoyloxycarbonyle, amino, alcoylamino ou dialcoylamino), ou représentent des radicaux benzyle ou hétérocyclyle saturé ou insaturé contenant 5 ou 6 chaînons et 1 à 3 hétéroatomes, ou pour lequel R₁ et R₂ forment avec l'atome d'azote auquel ils sont rattachés un hétérocycle de 4 à 6 chaînons pouvant contenir un autre hétéroatome choisi parmi l'azote, l'oxygène ou le soufre et éventuellement substitué par alcoyle, phényle ou benzyle,
      · soit un radical de formule générale : 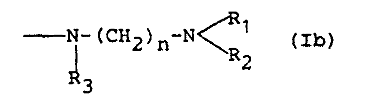 pour lequel R₁ et R₂ sont définis comme ci-dessus, R₃ représente un atome d'hydrogène ou un radical alcoyle et n est un nombre entier de 2 à 4,
R' représente un radical

   -CH₂-CH=CH-CH₂-R₄ (Ic) ou -CH₂-S-R'₄ (Id)

   pour lequel R₄ représente un radical alcoylthio, aminoalcoylthio, alcoylaminoalcoylthio, dialcoylaminoalcoylthio, pyrimidinylthio, thiazolylthio, N-alcoylimidazolylthio, hydroxyalcoylphénylthio, hydroxyalcolylphényloxy, nitrophénylamino, 2-oxopyrimidin-1-yle, et R'₄ représente un radical alcoyle, et
R" représente un atome d'hydrogène ou un radical hydroxy,
sous réserve que R et R" ne soient pas simultanément un atome d'hydrogène, et étant entendu que les portions ou radicaux alcoyle définis ci-dessus sont droits ou ramifiés et contiennent 1 à 4 atomes de carbone,
ainsi que leurs sels pharmaceutiquement acceptables, lorsqu'ils existent, sont particulièrement intéressants du fait de leur puissante activité.

Dans la formule générale (I), lorsque R₁ et/ou R₂ représentent hétérocyclyle, celui-ci peut être avantageusement choisi parmi pyridyle, tétrahydropyridyle, pipéridyle, imidazolyle, oxazolyle, thiazolyle.

Lorsque R₁ et R₂ forment hétérocyclyle avec l'atome d'azote auquel ils sont attachés, à titre d'exemple, le radical hétérocyclyle peut être choisi parmi azétidinyle, pipéridyle, pipérazinyle, N-méthyl pipérazinyle, N-phényl pipérazinyle, N-benzyl pipérazinyle, pyridyle, imidazolyle, morpholino, thiomorpholino, tétrahydropyridyle, méthyl tétrahydropyridyle (par exemple 4-méthyl tétrahydropyridyle), phényl tétrahydropyridyle (par exemple 4-phényl tétrahydropyridyle).

Selon la présente invention les produits de formule générale (I) pour lesquels R' est un radical de formule (Ic) peuvent être obtenus à partir de la 8'-bromo 3'-acétoxy cyclosporine de formule générale : 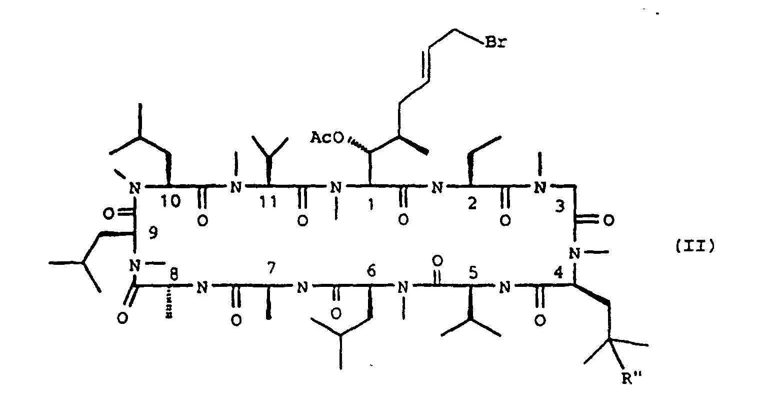 dans laquelle R" est défini comme précédemment, par action d'un thiol de formule générale :

HS-R"₄ (IIIa)

dans laquelle R"₄ représente un radical alcoyle, aminoalcoyle, alcoylaminoalcoyle, dialcoylaminoalcoyle, pyridinyle, thiazolyle, N-alcoylimidazolyle, hydroxyalcoylphényle, ou un phénol de formule générale :

HO-R'''₄ (IIIb)

dans laquelle R'''₄ représente un radical hydroxyalcoylphényle ou une amine de formule générale :

H₂N-R''''₄ (IIIc)

dans laquelle R''''₄ représente un radical nitrophényle ou de la 2-oxopyrimidine, en opérant en milieu basique, puis élimination du radical acétyle protecteur d'hydroxy, suivie le cas échéant, de l'opération subséquente d'hydroxylation en position 4' du résidu [MeLeu]⁴ lorsque l'on veut obtenir un dérivé pour lequel R" est hydroxy et lorsque l'on a effectué la réaction au départ d'un dérivé de cyclosporine pour lequel R" est un atome d'hydrogène et/ou suivi de l'opération subséquente de substitution de la chaîne de formule générale (Ia) lorsque l'on veut obtenir un dérivé de cyclosporine pour lequel R est autre que l'atome d'hydrogène.

La réaction s'effectue notamment en présence d'un thiolate, d'un phénolate ou d'un amidure alcalin (de préférence de sodium), dans un solvant tel qu'une cétone (acétone par exemple) ou un amide (diméthylformamide par exemple), à une température entre 20 et 45°C.

Le dérivé bromé de formule générale (II) pour lequel R" est un atome d'hydrogène est décrit dans J. Org. Chem. 57, 2689, (1992). Le dérivé correspondant pour lequel R" est un radical hydroxy peut être préparé par analogie.

Selon la présente invention les produits de formule générale (I) pour lesquels R' est un radical de formule (Id) peuvent être obtenus à partir d'un anhydride mixte de l'acide de formule générale : 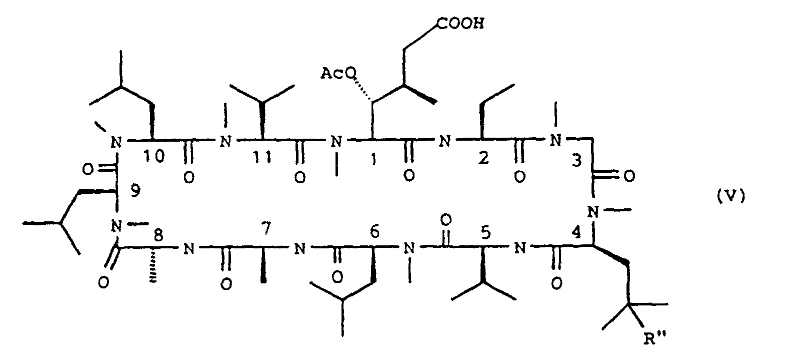 dans laquelle R" est défini comme précédemment et Ac représente un radical acétyle, tout d'abord transformé en ester thiohydroxamique par action d'un sel de la N-hydroxy-2-thiopyridine à l'abri de la lumière, puis transformé photochimiquement, par irradiation, en présence d'un disulfure de formule générale :

R'₄-S-S-R'₄ (IV)

dans laquelle R'₄ est défini comme précédemment, et débarrassé du radical acétyle protecteur de l'hydroxy en position -3' et enfin suivie le cas échéant, de l'opération subséquente d'hydroxylation en position 4' du résidu [MeLeu]⁴ lorsque l'on veut obtenir un dérivé pour lequel R" est hydroxy et lorsque l'on a effectué la réaction au départ d'un dérivé de cyclosporine pour lequel R" est un atome d'hydrogène et/ou suivi de l'opération subséquente de substitution de la chaîne de formule générale (Ia) lorsque l'on veut obtenir un dérivé de cyclosporine pour lequel R est autre que l'atome d'hydrogène.

La réaction s'effectue par analogie avec la méthode décrite par D. Barton dans Tetrahedron 43, 4297 (1987), dans un solvant organique tel qu'un solvant chloré (chloroforme, dichlorométhane, dichloroéthane par exemple) ou un éther (tétrahydrofurane, dioxane par exemple) à une température de 5 à 10°C dans l'obscurité puis par irradiation à la lumière à une température comprise entre 10 et 30°C. De préférence on utilise le sel de sodium de la N-hydroxy-2-thiopyridine.

L'acide de formule générale (V) dont la fonction hydroxy est protégée a été décrit par P. Paprika et coll., Bioconjugate Chem., 3, 32-36 (1992).

L'hydroxylation en position 4' du résidu [MeLeu]⁴ en un dérivé 4'-hydroxy-MeLeu s'effectue selon ou par analogie avec la méthode décrite dans la demande de brevet européen EP 484281.

La substitution de la chaîne de formule générale (Ia), en position -3, s'effectue par action d'un disulfure de formule générale

R°-Alk-S-S-Alk-R° (VI)

dans laquelle R° et Alk sont définis comme ci-dessus et dont le cas échéant les fonctions pouvant interférer avec la réaction ont été préalablement protégées, sur une forme activée d'un dérivé de cyclosporine de formule : 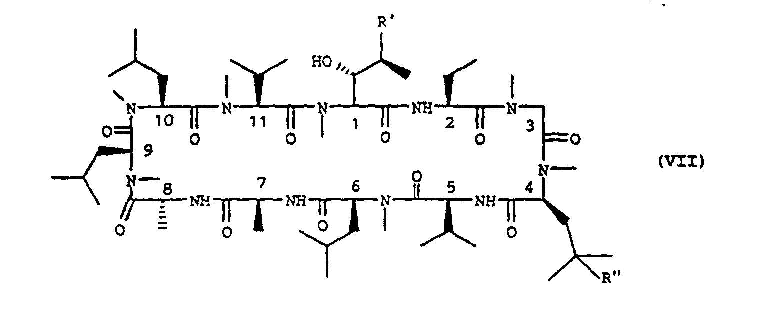 dans laquelle R' et R" sont définis comme précédemment, suivie de l'élimination le cas échéant du/des radicaux protecteurs.

On entend par forme activée de la cyclosporine de formule générale (II), une forme activée sur la sarcosine en position 3. Cette forme activée est préparée de préférence in situ. L'activation s'effectue généralement sous atmosphère inerte, par traitement par un dérivé organométallique (lithien notamment, comme le n-butyllithium, le diisopropyl amidure de lithium ou un mélange par exemple). Il est également possible de préparer la forme activée de la cyclosporine de formule générale (VII) dans l'ammoniac liquide en présence d'un amidure alcalin (sodium, lithium par exemple), à une température comprise entre -32 et -38°C, dans un éther (notamment tétrahydrofurane, t.butyléthyléther ou un mélange).

L'addition du disulfure de formule générale (VI), s'effectue avantageusement dans un solvant organique tel qu'un hydrocarbure (hexane par exemple) ou un éther (diéthyléther, tétrahydrofurane ou t.butylméthyléther par exemple) à une température comprise entre -78 et 0°C. Il est parfois préférable d'opérer sous azote.

Lorsque les substituants du radical R° peuvent interférer avec la réaction, il est préférable de les protéger préalablement par des radicaux compatibles et pouvant être mis en place et éliminés sans toucher au reste de la molécule. De plus les radicaux hydroxy présents sur la cyclosporine peuvent être éventuellement protégés par tout groupement qui n'interfère pas avec la réaction.

A titre d'exemple les groupements protecteurs peuvent être choisis parmi les radicaux décrits par T.W. GREENE, Protective Groups in Organic Synthesis, J. Wiley-Interscience Publication (1991) ou par Mc omie, Protective Groups in Organic Chemistry, Plenum Press (1973).

Lorsque R'' est un radical hydroxy, le dérivé de formule générale (VII) peut être préparé comme décrit dans la demande de brevet européen EP 484281 puis modification de la chaîne en position 1 comme décrit précédemment.

Les nouveaux dérivés de la cyclosporine de formule générale (I) peuvent être purifiés le cas échéant par des méthodes physiques telles que la cristallisation ou la chromatographie.

Les dérivés de la cyclosporine selon l'invention pour lesquels R° est carboxy peuvent être transformés en sels métalliques ou en sels d'addition avec une base azotée selon les méthodes connues en soi. Ces sels peuvent être obtenus par action d'une base métallique (par exemple alcaline ou alcalino-terreuse), de l'ammoniaque ou d'une amine sur un produit selon l'invention, dans un solvant approprié tel que l'eau ou un alcool. Le sel formé précipite après concentration éventuelle de la solution ; il est séparé par filtration.

Comme exemples de sels pharmaceutiquement acceptables peuvent être cités les sels avec les métaux alcalins (sodium, potassium, lithium) ou avec les métaux alcalino-terreux (magnésium, calcium), le sel d'ammonium, les sels de bases azotées (éthanolamine, diéthanolamine, triméthylamine, triéthylamine, méthylamine, propylamine, diisopropylamine, N,N-diméthyléthanolamine, benzylamine, dicyclohexylamine, N-benzylphénéthylamine, N,N'-dibenzyléthylènediamine, diphénylènediamine, benzhydrylamine, quinine, choline, arginine, lysine, leucine, dibenzylamine).

Les dérivés de la cyclosporine selon l'invention pour lesquels R° est NR₁R₂ peuvent être transformés en sels d'addition avec les acides, par les méthodes connues. Il est entendu que ces sels entrent aussi dans le cadre de la présente invention.

Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, p.toluènesulfonates, iséthionates, ou avec des dérivés de substitution de ces composés).

Les nouveaux dérivés de cyclosporine selon la présente invention sont particulièrement utiles pour la prophylaxie et le traitement des affections à rétrovirus et plus particulièrement du SIDA et de syndromes associés. Par prophylaxie on sous-entend notamment le traitement des sujets qui ont été exposés aux virus VIHs, en particulier les séropositifs asymptomatiques qui présentent le risque de développer la maladie dans les mois ou les années à venir après la primoinfection.

Les produits selon l'invention manifestent une activité anti-rétrovirus à des concentrations dépourvues d'effet cytotoxique ou cytostatique.

L'activité des produits de formule générale (I) a été mise en évidence dans les techniques décrites par Pauwells et Coll., J. Virol. Meth., 20, 309 (1988) et par O. Schwatz et Coll., AIDS Research and Human Retroviruses, 4(6), 441-48 (1988) et citées par J.F. Mayaux et al. Proc. Nat. Acad. Sci. USA, 91, 3564-68 (1994). L'inhibition de l'effet cytopathogène du virus par les produits selon l'invention s'exerce à des valeurs comprises entre 2 et 25 exprimées par le rapport entre la CE₅₀ de la Cyclosporine A et la CE₅₀ du produit étudié.

D'un intérêt particulier sont les produits de formule générale (I) pour lesquels :
R est un atome d'hydrogène ou un radical de structure (Ia) pour lequel
   - Alk-R° représente un radical méthyle, ou bien
   - Alk représente un radical alcoylène contenant 2 à 6 atomes de carbone en chaîne droite ou ramifiée et R° représente un radical -NR₁R₂ pour lequel R₁ et R₂ identiques ou différents représentent des radicaux alcoyle,
R' représente un radical

   -CH₂-CH=CH-CH₂-R₄ (Ic) ou - CH₂-S-R'₄ (Id)

   pour lequel R₄ représente un radical alcoylthio, et R'₄ représente un radical alcoyle, et
R" représente un atome d'hydrogène ou un radical hydroxy,
étant entendu que les portions ou radicaux alcoyle définis ci-dessus sont droits ou ramifiés et contiennent 1 à 4 atomes de carbone, et sous réserve que R et R" ne soient pas simultanément un atome d'hydrogène, ainsi que leurs sels pharmaceutiquement acceptables lorsqu'ils existent.

Selon un aspect encore plus préférentiel de l'invention, les dérivés de cyclosporine de formule générale (I) pour lesquels :
R est un radical de structure (Ia) pour lequel
   - Alk-R° représente un radical méthyle, ou bien
   - Alk représente un radical éthylène et R° représente un radical -NR₁R₂ pour lequel R₁ et R₂ identiques ou différents représentent des radicaux alcoyle contenant 1 ou 2 atomes de carbone,
R' représente un radical

   -CH₂-CH=CH-CH₂-R₄ (Ic) ou - CH₂-S-R'₄ (Id)

   pour lequel R₄ représente un radical méthylthio, et R'₄ représente un radical méthyle, et
R" représente un atome d'hydrogène ou un radical hydroxy,
ou bien R est un atome d'hydrogène, R' est défini comme précédemment et R" est un radical hydroxy,
ainsi que leurs sels pharmaceutiquement acceptables lorsqu'ils existent.

Et parmi ces produits notamment les dérivés de cyclosporine ci-après :
- [(3R,4R)-3-hydroxy-5-méthylthio-N-méthyl-L-leucine]¹[(2R)-méthyl-thiosarcosine]³-cyclosporine A ;
- [(3R,4R)-3-hydroxy-5-méthylthio-N-méthyl-L-leucine]¹[(2R)-(2'-diéthylamino)-éthylthiosarcosine]³-cyclosporine A ;
- [(3R,4R)-3-hydroxy-5-méthylthio-N-méthyl-L-leucine]¹[4-hydroxy-N-méthylleucine]⁴-cyclosporine A ;
- [((4R)-4-(4-méthylthio-butèn-2-yl-(E))-N,4-diméthylthréonine]¹-[(2R)-(2'-diéthylamino)-éthylthiosarcosine]³-cyclosporine A ;
- [(3R,4R)-3-hydroxy-5-méthylthio-N-méthyl-L-leucine]¹ [(2R)-(2'-di-méthylamino)-éthylthiosarcosine]³ [4'-hydroxy-N-méthyl-L-leucine]⁴ cyclosporine A ;
ainsi que leurs sels pharmaceutiquement acceptables lorsqu'ils existent.

Les exemples suivants donnés à titre non limitatif illustrent la présente invention.

### Exemple 1

La [(3R,4R)-3-hydroxy-5-méthylthio-N-méthyl-L-leucine]¹[(2R)-méthyl-thiosarcosine]³-cyclosporine A est préparée selon la méthode suivante : à une solution de 3,3 cm³ de diisopropylamine (préalablement distillée sur hydrure de calcium) et de 16,6 cm³ de diméthyl-1,3-(1H)-tétrahydro-3,4,5,6-pyrimidin-2-one dans 33 cm³ de tétrahydrofuranne (préalablement distillé sur sodium) refroidie à une température voisine de -70°C et sous azote, on ajoute en 15 minutes, 14,8 cm³ d'une solution de n-butyllithium 1,6 M dans l'hexane en maintenant la température à -70°C. Le mélange est agité à 0°C pendant 20 minutes, puis refroidi à une température voisine de -78°C. A la solution ainsi obtenue on ajoute une solution de 1,9 g de [(3R,4R)-3-hydroxy-5-méthylthio-N-méthyl-L-leucine]¹-cyclosporine A et de 14,4 cm³ de diméthyl-1,3-(1H)-tétrahydro-3,4,5,6-pyrimidin-2-one dans 27 cm³ de tétrahydrofuranne préalablement refroidie à une température voisine de -78°C en maintenant la température vers -68°C. Le mélange résultant est agité à une température voisine de -70°C pendant 20 minutes, puis 2,8 cm³ de disulfure de diméthyle sont ajoutés en 5 minutes en maintenant la température vers -70°C. Le mélange est agité à une température voisine de -78°C pendant 2 heures puis à 0°C pendant 20 heures. On verse lentement sur le mélange réactionnel 70 cm³ d'une solution d'acide chlorhydrique 1N puis 100 cm³ d'éther de diéthyle. La phase organique est décantée, lavés avec 100 cm³ au total d'eau distillée, séchée sur sulfate de sodium, filtrée et concentrée sous pression réduite (2,7 kPa) à une température voisine de 40°C. Le solide obtenu (2,1 g) est purifié par chromatographie sur colonne de silice (0,04-0,063 mm) en éluant avec de l'acétate d'éthyle. Les fractions contenant le produit attendu sont réunies et concentrées sous pression réduite (2,7 kPa) à une température voisine de 40°C pour donner un solide qui est concrété dans 20 cm³ de pentane. Après filtration, on obtient 110 mg de [3R,4R)-3-hydroxy-5-méthylthio-N-méthyl-L-leucine]¹[(2R)-méthylthiosarcosine]³-cyclosporine A sous la forme d'un solide blanc fondant vers 150°C.

Spectre de R.M.N. ¹ H (400 MHz, CDCl₃, δ en ppm) : 1,27 (d, J = 7 Hz, 3H : CH₃ 8β) ; 1,36 (d, J = 7,5 Hz, 3H : CH₃ 7β) ; 2,04 (s, 3H : SCH₃ 1ε) ; 2,15 (s, 3H : SCH₃ 3β) ; 2,30 et 2,83 (2 dd, respectivement J = 13 et 8,5 Hz et J = 13 et 3,5 Hz, 1H chacun : CH₂S 1δ) ; 2,44 (mt, 1H : CH 5β) ; 2,70 - 2,72 - 3,11 - 3,28 - 3,44 et 3,53 (6 s, respectivement 3H - 3H - 6H - 3H - 3H et 3H : 7 NCH₃) ; 3,84 (mt, 1H : CH 1β) ; 4,02 (d, J = 6 Hz, 1H : OH en 1β) ; 4,55 (mt, 1H : CH 7α) ; 4,66 (t, J = 9 Hz, 1H : CH 5α) ; 4,84 (mt, 1H : CH 8α) ; 4,96 (dd, J = 9,5 et 6 Hz, 1H : CH α d'une leucine) ; de 5,00 à 5,15 (mt, 2H : CH 2a et CH α d'une leucine) ; 5,17 (d, J = 11 Hz, 1H : CH 11α) ; 5,28 (dd, J = 12 et 4 Hz, 1H : CH α d'une leucine) ; 5,49 (d, J = 6 Hz, 1H : CH 1α) ; 5,72 (dd, J = 11 et 4 Hz, 1H : CH α d'une leucine) ; 5,78 (s, 1H : CH 3α) ; 7,17 (d, J = 8 Hz, 1H : CONH en 8) ; 7,34 (d, J = 9 Hz, 1H : CONH en 5) ; 7,66 (d, J = 7,5 Hz, 1H : CONH en 7) ; 7,93 (d, J =10 Hz, 1H : CONH en 2).

La [(3R,4R)-3-acétyloxy-5-méthylthio-N-méthyl-L-leucine]¹-cyclosporine A peut être préparée à l'abri de la lumière et sous atmosphère inerte, selon la méthode suivante :

A une solution de 12,5 g [(3R,4R)-3-acétyloxy-5-carboxy-N-méthyl-L-leucine]¹-cyclosporine A dans 100 cm³ de 1,2-dichloroéthane, refroidie à une température voisine de -15°C, on ajoute successivement en 5 min. 1,2 cm³ de N-méthylmorpholine (préalablement distillée) et en 10 min., 1,4 cm³ de chloroformiate d'isobutyle (préalablement distillé). Le mélange réactionnel est agité durant 90 min. à une température voisine de -15°C puis une solution de 1,5 g de sel de sodium de l'oxyde de la 2-mercaptopyridine et de 1,7 cm³ de triéthylamine dans 25 cm³ de 1,2-dichloroéthane est ajoutée en 10 minutes. Le mélange réactionnel est agité à l'abri de la lumière durant 17 heures à une température voisine de -20°C. On ajoute ensuite en 5 min., 8,9 cm³ de disulfure de diméthyle (préalablement distillée), puis on irradie durant 3 heures à l'aide de deux lampes au tungstène de 60 W en maintenant la température entre 0 et 10°C. Le mélange résultant est alors concentré sous pression réduite (2,7 kPa) à une température voisine de 40°C. Le résidu est repris par 200 cm³ d'éther de diéthylique, 100 cm³ d'eau distillée et 20 cm³ d'une solution N d'acide chlorhydrique. La phase organique est décantée puis lavée successivement avec 150 cm³ au total d'eau distillée et 25 cm³ d'une solution aqueuse molaire d'éthanolamine puis 125 cm³ au total d'eau distillée. La phase organique est séchée sur sulfate de magnésium, filtrée et concentrée sous pression réduite (2,7 kPa) à une température voisine de 40°C. La meringue jaune obtenue (10,5 g) est purifiée par chromatographie sur colonne de silice (0,04-0,063 mm) en éluant avec de l'acétate d'éthyle. Les fractions contenant le produit attendu sont réunies et concentrées sous pression réduite (2,7 kPa) à une température voisine de 40°C pour donner 4,0 g de [(3R,4R)-3-acétyloxy-5-méthylthio-N-méthyl-L-leucine]¹-cyclosporine A sous la forme d'une meringue blanche (Rf = 0,31; chromatographie sur couche mince de silice; éluant : acétate d'éthyle).

Spectre de R.M.N. ¹ H (400 MHz, CDCl₃, δ en ppm) : 1,28 (d, J = 7 Hz, 3H : CH₃ 8β) ; 1,32 (d, J = 7,5 Hz, 3H : CH₃ 7β) ; 1,92 (s, 3H : COCH₃ en 1β) ; 2,00 (s, 3H : SCH₃ 1ε) ; 2,34 et 2,50 (2 dd, respectivement J = 13 et 5 Hz et J = 13 et 7 Hz, 1H chacun : SCH₂ 1δ) ; 2,45 (mt, 1H : CH 5β) ; 2,65 - 2,68 - 3,05 - 3,20 - 3,26 - 3,29 et 3,45 (7 s, 3H chacun : 7 NCH₃) ; 3,14 et 4,66 (2 d, J = 14 Hz, 1H chacun : CH₂ 3α) ; 4,42 (mt, 1H : CH 7α) ; 4,75 (t, J = 9,5 Hz, 1H : CH 5α) ; 4,86 (mt, 1H : CH 8α) ; 4,96 (mt, 1H : CH 2 α) ; 5,00 (d, J = 11 Hz, 1H : CH 11α) ; 5,12 (dd, J = 7 et 5 Hz, 1H : CH α d'une leucine) ; 5,35 (dd, J = 12 et 4 Hz, 1H : CH α d'une leucine) ; de 5,40 à 5,60 (mt, 3H : CH α d'une leucine - CH 1α et CH 1β) ; 5,68 (dd, J = 11 et 4 Hz, 1H : CH α d'une leucine) ; 7,51 (d, J = 8 Hz, 1H : CONH en 8) ; 7,61 (d, J = 9,5 Hz, 1H : CONH en 5) ; 8,07 (d, J = 7,5 Hz, 1H : CONH en 7) ; 8,53 (d, J = 10 Hz, 1H : CONH en 2).

[(3R,4R)-3-acétyloxy-5-carboxy-N-méthyl-L-leucine]¹-cyclosporine A peut être préparé selon P. Paprika et al. Bioconjugate Chem. 3, 32-36 (1992).

[(3R,4R)-3-hydroxy-5-méthylthio-N-méthyl-L-leucine]¹-cyclosporine A peut être préparée selon la méthode suivante :

7,2 cm³ d'une solution 0,5 M en méthylate de sodium sont ajoutés à une solution de 310 mg de chlorhydrate de guanidine dans 20 cm³ de méthanol. La solution obtenue est ajoutée à une solution de 3,75 g [(3R,4R)-3-acétyloxy-5-méthylthio-N-méthyl-L-leucine]¹-cyclosporine A dans 130 cm³ de méthanol. Le mélange résultant est agité à une température voisine de 20°C pendant 48 heures puis le mélange est concentré concentrée sous pression réduite (2,7 kPa) à une température voisine de 40°C. Le résidu est repris par 100 cm³ d'éther de diéthylique. La phase organique est lavée par 150 cm³ au total d'eau distillée, séchée sur sulfate de sodium, filtrée et concentrée sous pression réduite (2,7 kPa) à une température voisine de 40°C. Le solide obtenu (3,5 g) est purifié par chromatographie sur colonne de silice (0,04-0,063 mm) en éluant avec de l'acétate d'éthyle. Les fractions contenant le produit attendu sont réunies et concentrées sous pression réduite (2,7 kPa) à une température voisine de 40°C pour donner 2,5 g de [(3R,4R)-3-hydroxy-5-méthylthio-N-méthyl-L-leucine]¹-cyclosporine A sous la forme d'une meringue blanche (Rf = 0,32 ; chromatographie sur couche mince de silice ; éluant : acétate d'éthyle).

Spectre de R.M.N. ¹ H (400 MHz, CDCl₃, δ en ppm) : 1,26 (d, J = 7 Hz, 3H : CH₃ 8β) ; 1,36 (d, J = 7,5 Hz, 3H : CH₃ 7β) ; 1,90 (mt, 1H : CH en 1γ) ; 2,04 (s, 3H : SCH₃ 1ε) ; 2,31 et 2,87 (2 dd, respectivement J = 13 et 9 Hz et J = 13 et 3,5 Hz, 1H chacun : CH₂S 1δ) ; 2,43 (mt, 1H : CH 5β) ; 2,70 - 2,72 - 3,11 - 3,26 - 3,39 et 3,52 (6 s, respectivement 3H - 3H - 6H - 3H - 3H et 3H : 7 NCH₃) ; 3,20 et 4,74 (2d, J = 14,5 Hz, 1H chacun : CH₂ 3α) ; 3,85 (mt, 1H : CH 1b) ; 4,21 (d, J = 6 Hz, 1H : OH en 1β) ; 4,54 (mt, 1H : CH 7α) ; 4,65 (t, J = 9 Hz, 1H : CH 5α) ; 4,83 (mt, 1H : CH 8α) ; 4,97 (dd, J = 9,5 et 6 Hz, 1H : CH α d'une leucine) ; de 5,00 à 5,15 (mt, 2H : CH 2α et CH α d'une leucine) ; 5,16 (d, J = 11 Hz, 1H : CH 11α) ; 5,34 (dd, J = 12 et 4 Hz, 1H : CH α d'une leucine) ; 5,48 (d, J = 6 Hz, 1H : CH 1α) ; 5,72 (dd, J = 11 et 4 Hz, 1H : CH α d'une leucine) ; 7,17 (d, J = 8 Hz, 1H : CONH en 8) ; 7,48 (d, J = 9 Hz, 1H : CONH en 5) ; 7,67 (d, J = 7,5 Hz, 1H : CONH en 7) ; 7,97 (d, J = 10 Hz, 1H : CONH en 2).

### Exemple 2

La [(3R,4R)-3-hydroxy-5-méthylthio-N-méthyl-L-leucine]¹[(2R)-(2'-diéthylamino)-éthylthiosarcosine]³-cyclosporine A est préparée en opérant comme à l'exemple 1, mais à partir de 1,1 g de [(3R,4R)-3-hydroxy-5-méthylthio-N-méthyl-L-leucine]¹-cyclosporine A et 5,2 g de disulfure de 2-(diéthylamino)éthyle, puis en purifiant comme décrit ci-après.

La phase organique est décantée, lavé avec 90 cm³ au total d'eau distillée, séchée sur sulfate de sodium, filtrée et concentrée sous pression réduite (2,7 kPa) à une température voisine de 40°C. Le résidu huileux obtenu (6,2 g) est trituré avec 150 cm³ de pentane pour conduire à 700 mg d'un solide blanc qui est purifié par chromatographie sur colonne de silice (0,04-0,063 mm) en éluant avec un mélange d'acétate d'éthyle et de méthanol (4:1 en volumes). Les fractions contenant le produit attendu sont réunies et concentrées sous pression réduite (2,7 kPa) à une température voisine de 40°C pour donner 300 mg d'un solide blanc qui est purifié par une seconde chromatographie sur colonne de silice (0,04-0,063 mm) en éluant avec un mélange d'acétate d'éthyle, de 1-butanol, d'éthanol et d'eau (8 ; 6 ; 3 ; 3 en volumes). Les fractions contenant le produit attendu sont réunies et concentrées sous pression réduite (2,7 kPa) à une température voisine de 40°C pour donner 150 mg d'un solide blanc qui est trituré avec 5 cm³ de pentane. Après filtration, on obtient 33 mg de [(3R,4R)-3-hydroxy-5-méthylthio-N-méthyl-L-leucine]¹ [(2R)-(2'-diéthylamino)éthylthiosarcosine]³-cyclosporine A sous la forme d'un solide blanc fondant vers 140°C.

Spectre de R.M.N. ¹ H (400 MHz, CDCl₃, δ en ppm). Nous observons deux conformères dans les proportions 75% et 25% . 1,37 (d, J = 7,5 Hz, 3H : CH₃ 7β) ; 2,00 et 2,03 (s, 3H en totalité : SCH₃ 1δ) ; de 2,30 à 2,90 (mt : les 11H correspondant aux SCH₂CH₂N(CH₂)₂ du 2-diéthylaminoéthylthio en 3α - SCH₂ 1δ et CH 5β) ; 3,80 (mt, 1H : CH 1b) ; 3,97 (d large, J = 6 Hz, 1H : OH en 1β) ; 4,54 et de 4,59 à 4,70 (2 mts, 1H en totalité : CH 7α) ; de 4,60 à 4,70 (mt, 1H : CH 5α) ; 4,84 (mt, 1H : CH 8α) ; de 4,85 à 5,45 (mt, 4H : CH 11α et CH α de trois leucines) ; 5,49 et 5,66 (2 d, respectivement J = 6 et J = 9 Hz, 1H en totalité : CH 1a) ; 5,72 (dd, J = 10,5 et 4 Hz, 1H : CH α d'une leucine) ; 5,79 et 5,98 (2 s, 1H en totalité : CH 3α) ; 7,05 et 7,17 (2 d, J = 8 Hz, 1H en totalité : CONH en 8) ; 7,32 et 7,35 (2 d, J = 9 Hz, 1H en totalité : CONH en 5) ; 7,42 et 7,76 (2 d, J = 7,5 Hz, 1H en totalité : CONH en 7) ; 7,94 (d large, J = 10 Hz, 1H : CONH en 2).

### Exemple 3

La [(3R,4R)-3-hydroxy-5-méthylthio-N-méthyl-L-leucine]¹[4-hydroxy-N-méthylleucine]⁴-cyclosporine A est préparée par hydroxylation avec la souche Sebekia benihana du produit tel qu'obtenu à l'exemple 1 sur lequel la chaîne en position -1 a été initialement modifiée.

Deux erlenmeyers de 250 cm³, contenant 50 cm³ de milieu A (peptone: 10 g ; extrait de levure: 5 g ; amidon : 10 g ; glucose : 5 g ; agar : 1 g ; eau qsp : 1000 cm³ ; pH ajusté à 7,2 ; stérilisation à 121°C pendant 25 minutes) sont ensemencés à 2 % à partir de deux ampoules liquides congelées de la souche Sebekia benihana puis sont incubés sous agitation à 220 tours/minute pendant 72 heures à une température voisine de 28°C. Ces quatre erlenmeyers constituent les erlenmeyers inoculum.

Sept erlenmeyers de 250 cm³ contenant 50 cm³ de milieu B (glucose : 10 g ; amidon soluble: 10 g ; extrait de levure : 2,5 g ; farine de soja : 12,5 g ; dextrine : 10 g ; dihydrogénophosphate de potassium : 0,12 g ; sulfate de magnésium heptahydrate : 0,10 g ; hydrogéno-phosphate dipotassique : 0,25 g ; chlorure de calcium dihydrate : 0,05 g ; [1 cm³ d'une solution contenant H₃BO₃ : 0,1g ; FeSO₄, 7H₂O : 5 g ; KI: 0,05 g ; CaCl₂, 6H₂O : 2 g ; CuSO₄, 5H₂O: 0,2g ; MnCl₂, 4H2O : 2 g ; ZnSO₄, 7H₂O : 4 g ; (NH₄)₂Mo₇O₂₄ : 0,2g; H₂SO₄ 97 % : 1 cm³ ; H₂O qsp : 1000 cm³] ; eau qsp : 1000 cm³ : pH ajusté à 7,2-7,5 ; stérilisation à 121°C pendant 25 minutes) sont ensemencés à 4 % à partir des erlenmeyers inoculum, puis sont incubés sous agitation à 220 tours/minute pendant 48 heures à une température voisine de 28°C avant ajout de la [(3R,4R)-3-hydroxy-5-méthylthio-N-méthyl-L-leucine]¹-cyclosporine A. Ces erlenmeyers constituent les erlenmeyers de production

Une solution de 105 mg de [(3R,4R)-3-hydroxy-5-méthylthio-N-méthyl-L-leucine]¹cyclosporine A dans 3,5 cm³ d'éthanol est préparée extemporanément, puis filtrée sur filtre Milipore 0,2 µ. On ajoute stérilement dans chacun des erlenmeyers de production 0,5 cm³ de la solution mère de [(3R,4R)-3-hydroxy-5-méthylthio-N-méthyl-L-leucine]¹ [4-hydroxy-N-méthylleucine]⁴-cyclosporine A. Les erlenmeyers sont incubés sous agitation à 220 tours/minute à une température voisine de 28°C. Après 72 heures, chaque erlenmeyer est extrait avec un mélange d'acétonitrile, de n-heptane et de méthyl-t-butyléther (2:1:1 en volumes). Les phases organiques intermédiaires sont réunies, concentrées sous pression réduite (2,7 kPa) à une température voisine de 40°C puis reprises dans 10 cm³ de méthanol. A la solution obtenue est ajouté 5 g de silice Sorbsil C60 (40-60 µm) (Prolabo) et le tout est concentré sous vide (2,7 kPa), puis séché 2 heures à l'étuve à une température voisine de 25°C. Le résidu est chromatographié sur silice Sorbsil C60 (40-60 µm) (Prolabo) éluée par du 1,2-dichloroéthane (débit 20 cm³/heure, fractions de 10 cm³). A partir de la fraction 8, on élue avec un mélange de 1,2-dichloroéthane et de méthanol (99:1 en volume) jusqu'à la fraction 15. Dès cette fraction, on utilise le système délution 1,2-dichloro-éthane/méthanol (49:1 en volume). Les fractions ne contenant que le produit attendu sont rassemblées et concentrées à sec sous pression réduite (2,7 kPa) à une température voisine de 40°C. Le résidu est repris par 2 cm³ d'acétate d'éthyle et 1 cm³ de cyclohexane puis on laisse 3 heures à une température voisine de 4°C. Le solide est ensuite filtré, lavé à l'heptane pour conduire à 33 mg de [(3R,4R)-3-hydroxy-5-méthylthio-N-méthyl-L-leucine]¹[4-hydroxy-N-méthyl-leucine]⁴-cyclosporine A sous forme d'un solide blanc.
Spectre de R.M.N. ¹ H (400 MHz, CDCl₃, δ en ppm) : 1,36 (d, J = 7,5 Hz, 3H : CH₃ 7β) ; 2,04 (s, 3H : SCH₃ 1ε) ; 2,08 et 2,37 (2 mts : les 2 H correspondant au CH₂ 4β) ; 2,40 et 2,83 (respectivement mt et dd, J = 13 et 3,5 Hz, 1H chacun : CH₂S 1δ) ; 2,44 (mt, 1H : CH 5β) ; 2,70 et 2,72 (2 s, 3H chacun : 2 NCH₃) ; 3,18 et 4,70 (2 d, J = 14 Hz, 1H chacun : CH₂ 3α) ; 3,86 (mt, 1H : CH 1β) ; 4,52 (mt, 1H : CH 7α) ; 4,68 (t, J = 9 Hz, 1H : CH 5α) ; 4,84 (mt, 1H : CH 8α) ; de 4,95 à 5,20 (mt, 3H : CH 2α et CH α de deux leucines) ; 5,16 (d, J = 11 Hz, 1H : CH 11α) ; 5,43 (d, J = 6 Hz, 1H : CH 1α) ; 5,58 (mt, 1H : CH 4α) ; 5,72 (dd, J = 11 et 4 Hz, 1H : CH α d'une leucine) ; 7,22 (d, J = 8 Hz, 1H : CONH en 8) ; 7,68 (d, J = 9 Hz, 1H : CONH en 5) ; 7,73 (d, J = 7,5 Hz, 1H : CONH en 7) ; 8,06 (d, J = 10 Hz, 1H : CONH en 2).

### Exemple 4

### [((4R)-4-(4-méthylthio-butèn-2-yl-(E))-N,4-diméthylthréonine]¹-[(2R)-méthylthiosarcosine]³-cyclosporine A :

Une solution de 1 g de [((4R)-4-(4-méthylthio-butèn-2-yl-(E))-N,4-diméthylthréonine]¹-cyclosporine A dans 40 cm³ de tétrahydrofuranne (THF) et 1,44 cm³ de diméthyl-1,3-(1H)-tétrahydro-3,4,5,6-pyrimidin-2-one (DMPU) est ajouté goutte à goutte à -70°C à 6 cm³ d'une solution de diisopropylamidure de lithium (LDA) 2N dans le THF/héptane, sous atmosphère d'argon. La température de la solution de couleur orange foncé est remontée à -30°C et maintenue ainsi pendant 30 minutes. Le mélange est ensuite refroidi à -70°C et 1,40 cm³ de disulfure de diméthyle sont ajoutés goutte à goutte. Après 1 heure la température est remontée à -10°C et maintenue ainsi pendant 2 heures. La réaction est arrêtée par l'ajout d'acide chlorhydrique 1N (HCl 1N), jusqu'à neutralité, et le mélange est extrait trois fois avec 30 cm³ d'ether éthylique. Les phases organiques réunies sont lavées deux fois par 20 cm³ d'eau et 20 cm³ d'une solution saturée de NaCl, puis sechées sur du sulfate de sodium anhydre, filtrés et concentrés à sec sous pression réduite (2,7 kPa) à une température voisine de 40°C. On obtient ainsi une huile orange qui est purifiée par chromatographie sur colonne de silice (0,04-0,063 mm) en éluant avec le mélange acétate d'éthyle/dichlorométhane 8/2. Les fractions contenant le produit attendu sont réunies et concentrées sous pression réduite (2,7 kPa) à une température voisine de 40°C pour donner 81 mg de [((4R)-4-(4-méthylthio-butèn-2-yl-(E))-N,4-diméthylthréonine]¹-[(2R)-méthylthiosarcosine]³-cyclosporine A sous forme d'un solide blanc qui fond vers 130°C.

Spectre de R.M.N. ¹ H (400 MHz, CDCl₃, δ en ppm) ; 1,28 (d, J = 7 Hz, 3H : CH₃ 8β) ; 1,35 (d, J = 7,5 Hz, 3H : CH₃ 7β) ; 1,99 (s, 3H : SCH₃ de la chaîne en 1) ; 2,15 (s, 3H : SCH₃ 3β) ; 2,45 (mt, 1H : CH 5β) ; 2,69 - 2,71 - 3,12 - 3,27 - 3,45 - et 3,52 (6 s, respectivement 3H - 3H - 6H - 3H - 3H et 3H : 7 NCH₃) ; 3,05 (d, J = 7,5 Hz, 2H : SCH₂ de la chaîne en 1) ; 3,53 (d large, J = 6 Hz, 1H : OH 1β) ; 3,78 (mt, 1H : CH 1β) ; 4,55 (mt, 1H : CH 7α) ; 4,67 (t, J = 9 Hz, 1H : CH 5α) ; 4,85 (mt, 1H : CH 8α) ; 4,97 (dd, J = 9 et 6 Hz, 1H : CH α d'une leucine) ; de 5,00 à 5,10 (mt, 3H : CH 2α - CHα d'une leucine et CH 11α) ; 5,23 (dd, J = 12 et 4 Hz, 1H : CH α d'une leucine) ; de 5,25 à 5,50 (mt, 2H : CH=CH) ; 5,52 (d, J = 6 Hz, 1H : CH 1α) ; 5,72 (dd, J = 11 et 4 Hz, 1H : CH α d'une leucine) ; 5,78 (s, 1H : CH 3α) ; 7,17 (d, J = 8 Hz, 1H : CONH en 8) ; 7,37 (d, J = 9 Hz, 1H : CONH en 5) ; 7,21 (d, J = 7,5 Hz, 1H : CONH en 7) ; 8,02 (d, J =10 Hz, 1H : CONH en 2).

La [((4R)-4-(4-méthylthio-butèn-2-yl-(E))-N,4-diméthylthréonine]¹-cyclosporine A est préparée selon la méthode suivante :

A une solution de méthanolate de sodium (préparé au préalable avec 7 mg de sodium metal dans 1 cm³ de méthanol) sont ajoutés 0,35 g de [((4R)-4-(4-méthylthio-butèn-2-yl-(E))-O-acétyl-N,4-diméthylthréonine]¹-cyclosporine A dans 5 cm³ de méthanol à 20°C sous argon. Le mélange jaune est laissé sous agitation pendant 24 heures puis acidifié à pH 6 par l'ajout d'acide acétique et concentré à sec. Le résidu est trituré dans l'éther éthylique et filtré pour donner un solide beige qui est purifiée par chromatographie sur colonne de silice (0,04-0,063 mm) en éluant avec de l'acétate d'éthyle saturé en eau. Les fractions contenant le produit attendu sont réunies et concentrées sous pression réduite (2,7 kPa) à une température voisine de 40°C pour donner 100 mg de [((4R)-4-(4-méthylthio-butèn-2-yl-(E))-N,4-diméthylthréonine]¹-cyclosporine A sous forme d'un solide blanc qui fond vers 148°C.

Spectre de R.M.N. ¹ H (400 MHz, CDCl₃, δ en ppm) ; 1,27 (d, J = 7 Hz, 3H : CH₃ 8β) ; 1,35 (d, J = 7,5 Hz, 3H : CH₃ 7β) ; 1,99 (s, 3H : SCH₃ de la chaîne en 1) ; 2,44 (mt, 1H : CH 5β) ; 2,70 - 2,72 - 3,12 - 3,27 - 3,30 - et 3,52 (6 s, respectivement 3H - 3H - 6H - 3H - 3H et 3H : 7 NCH₃) ; 3,06 (d, J = 7,5 Hz, 2H : SCH₂ de la chaîne en 1) ; 3,20 et 4,73 (2 d, J = 14,5 Hz, 1H chacun : CH₂ 3α) ; 3,72 (d, J = 6 Hz, 1H : OH en 1β) ; 3,81 (mt, 1H : CH en 1β) ; 4,54 (mt, 1H : CH 7α) ; 4,66 (t, J = 9 Hz, 1H : CH 5α) ; 4,84 (mt, 1H : CH 8α) ; de 4,90 à 5,10 (mt, 3H : CH 2α et CHα de deux leucines) ; 5,10 (d, J = 11 Hz, 1H : CH 11α) ; de 5,25 à 5,50 (mt, 3H : CHα d'une leucine et CH=CH) ; 5,51 (d, J = 6 Hz, 1H : CH 1α) ; 5,71 (dd, J = 11 et 4 Hz, 1H : CH α d'une leucine) ; 7,17 (d, J = 8 Hz, 1H : CONH en 8) ; 7,50 (d, J = 9 Hz, 1H : CONH en 5) ; 7,71 (d, J = 7,5 Hz, 1H : CONH en 7) ; 8,09 (d, J = 10 Hz, 1H : CONH en 2).

La [((4R)-4-(4-méthylthio-butèn-2-yl-(E))-O-acétyl-N,4-diméthylthréonine]'-cyclosporine A est préparée selon la méthode suivante :

A une solution de 0,4 g de [((4R)-4-(4-bromo-butèn-2-yl-(E))-O-acétyl-N,4-diméthylthréonine]¹-cyclosporine A dans 7 cm³ de diméthylformamide (DMF) à 20°C sous argon sont ajoutés 23 mg de méthanethiolate de sodium. Le mélange jaune est laissé sous agitation pendant 24 heures puis 10 cm3 d'eau sont ajoutés pour obtenir un précipité blanc qui est filtré et seché. On obtient ainsi 0,37 g de [((4R)-4-(4-méthylthio-butèn-2-yl-(E))-O-acétyl-N,4-diméthylthréonine]¹-cyclosporine A sous forme d'un solide blanc qui fond vers 137°C, utilisé tel quel pour la suite.

Spectre de R.M.N. ¹ H (400 MHz, CDCl₃, δ en ppm) ; 1,28 (d, J = 7 Hz, 3H : CH₃ 8β) ; 1,33 (d, J = 7,5 Hz, 3H : CH₃ 7β) ; 1,98 (s, 3H : SCH₃ de la chaîne en 1) ; 2,03 (s, 3H : OCOCH₃ en 1β) ; 2,43 (mt, 1H : CH 5β) ; 2,66 - 2,70 - 3,11 - 3,21 - 3,25 - 3,27 et 3,45 (7 s, 3H chacun : 7 NCH₃) ; de 2,90 à 3,10 (mt, 2H : SCH₂ de la chaîne en 1) ; 3,18 et 4,65 (2 d, J = 14 Hz, 1H chacun : CH₂ 3α) ; 4,41 (mt, 1H : CH 7α) ; 4,74 (t, J = 9,5 Hz, 1H : CH 5α) ; 4,85 (mt, 1H : CH 8α) ; de 4,85 à 5,00 (mt, 2H : CH 2a et CH 11α) ; 5,15 (dd, J = 8 et 6 Hz, 1H : CH α d'une leucine) ; de 5,15 à 5,40 (mt, 4H : CHα de deux leucines et CH=CH) ; 5,53 (s, 2H : CH 1α et CH 1β) ; 5,69 (dd, J = 11 et 4 Hz, 1H : CH α d'une leucine) ; 7,47 (d, J = 8 Hz, 1H : CONH en 8) ; 7,53 (d, J = 9,5 Hz, 1H : CONH en 5) ; 8,04 (d, J = 7,5 Hz, 1H : CONH en 7) ; 8,57 (d, J = 10 Hz, 1H : CONH en 2).

### Exemple 5

La [((4R)-4-(4-méthylthio-butèn-2-yl-(E))-N,4-diméthylthréonine]¹-[(2R)-(2'-diéthylamino)-éthylthiosarcosine]³-cyclosporine A est préparée selon la méthode décrite à l'exemple 4 à partir de 1 g de [((4R)-4-(4-méthylthio-butèn-2-yl-(E))-N,4-diméthylthréonine]¹-cyclosporine A en utilisant 3 g de disulfure de N,N-diéthylaminoéthanethiol. En fin d'addition du disulfure, le mélange est maintenu à -70°C pendant 1 heure, puis 16 heures à environ -10°C. Après rechauffement à 10°C, le mélange est acidifié à pH 2 avec de HCl 1N et extrait avec 60 cm³ d'ether éthylique, puis la phase organique est extraite trois fois avec 25 cm³ de HCl 1N. La phase aqueuse est basifié à pH = 8 avec du bicarbonate de sodium et extraite trois fois avec 50 cm³ d'ether éthylique. Les phases organiques réunies sont sechées sur du sulfate de sodium anhydre, filtrés et concentrés à sec sous pression réduite (2,7 kPa) à une température voisine de 40°C. L'huile épaisse orange obtenue est filtrée sur un gâteau de silice (0,04-0,063 mm) avec le mélange méthanol/dichlorométhane 9/1 puis, après rassemblement des fractions contenant le produit et évaporation du solvant, le résidu solide obtenu est purifiée par chromatographie sur colonne de silice (0,04-0,063 mm) en éluant avec le mélange acétate d'éthyle/butanol/ethanol/eau 4/3/1,5/0,5. Les fractions contenant le produit attendu sont réunies et concentrées sous pression réduite (2,7 kPa) à une température voisine de 40°C pour donner 37 mg de [((4R)-4-(4-méthylthio-butèn-2-yl-(E))-N,4-diméthylthréonine]¹-[(2R)-(2'-diéthylamino)éthylthiosarcosine]³-cyclosporine A sous forme d'un solide blanc qui fond vers 135°C.

Spectre de R.M.N. ¹ H (400 MHz, CDCl₃, δ en ppm) : 1,26 (d, J = 7 Hz, 3H : CH₃ 8β) ; 1,35 (d, J = 7,5 Hz, 3H : CH₃ 7β) ; 1,99 (s, 3H : SCH₃ de la chaîne en 1) ; 2,46 (mt, 1H : CH 5β) ; de 2,45 à 3,35 (mt : les 8H correspondant aux SCH₂CH₂N(CH₂)₂ du 2-diéthylaminoéthylthio en 3α) ; 3,04 (d, J = 7,5 Hz, 1H : CH₂S de la chaîne en 1) ; 2,68 - 2,72 - 3,11 - 3,13 - 3,25 - 3,44 et 3,51 (7 s, 3H chacun : 7 NCH₃) ; 3,58 (d large, J = 6 Hz, 1H : OH en 1β) ; 3,25 (mt, 1H : CH 1β) ; 4,55 (mt, 1H : CH 7α) ; 4,65 (t, J = 9 Hz, 1H : CH 5α) ; 4,84 (mt, 1H : CH 8α) ; 4,97 (dd, J = 9 et 6 Hz, 1H : CH α d'une leucine) ; de 5,00 à 5,15 (mt, 3H : CH 2α - CH α d'une leucine et CH 11α) ; 5,22 (dd, J = 12 et 4 Hz, 1H : CH α d'une leucine) ; de 5,20 à 5,55 (mt, 2H : CH=CH) ; 5,52 (d, J = 6 Hz, 1H : CH 1α) ; 5,72 (dd, J = 10,5 et 4 Hz, 1H : CH α d'une leucine) ; 5,98 (s, 1H : CH 3α) ; 7,17 (d, J = 8 Hz, 1H : CONH en 8) ; 7,36 (d, J = 9 Hz, 1H : CONH en 5) ; 7,70 (d, J = 7,5 Hz, 1H : CONH en 7) ; 8,04 (d, J = 10 Hz, 1H : CONH en 2).

### Exemple 6

### [(3R,4R)-3-hydroxy-5-méthylthio-N-méthyl-L-leucine]¹ [(2R)-(2'-diméthylamino)-éthylthiosarcosine]³ [4'-hydroxy-N-méthyl-L-leucine]⁴ cyclosporine A :

A 70 cm³ d'ammoniac maintenus à une température voisine de -33°C, on ajoute 50 mg de sodium métallique puis 10 mg de nitrate ferrique. Dès que la coloration bleue du milieu a disparu, 0,626 g de sodium métallique sont ajoutés en 15 minutes. Le mélange est agité à -33°C durant 90 minutes, puis une solution de 2,4 g de [(3R,4R)-3-hydroxy-5-méthylthio-N-méthyl-L-leucine]¹[4'-hydroxy-N-méthyl-L-leucine]⁴ cyclosporine A dans 80 cm³ de tert-butylméthyléther est ajoutée goutte à goutte pendant une durée approximative de 30 minutes suivie d'une solution de 1,63 g de disulfure de N,N-diméthylaminoéthanethiol dans 10 cm³ de tert-butylméthyléther. Le mélange réactionnel est agité à -33°C durant 30 minutes, puis 2,35 g de chlorure d'ammonium sont ajoutés par portions. Sous agitation, on laisse évaporer l'ammoniac en faisant passer la température du mélange de -33°C à 20°C en 12 heures. Le mélange réactionnel est dilué par 100 cm³ d'éther de diéthyle puis filtré. Le solide est rincé par 300 cm³ au total d'éther de diéthyle. Les phases organiques réunies sont concentrées sous pression réduite (2,7 kPa) à une température voisine de 30°C. L'huile résiduelle est agitée durant 48 heures à une température voisine de 20°C avec 200 cm³ de pentane. Le solide formé est filtré puis rincé avec 150 cm³ au total de pentane. Le solide est purifié par chromatographie sur une colonne de silice (0,020 - 0,045 mm) éluée par un mélange d'acétate d'éthyle et de méthanol 9:1 (en volumes) en recueillant des fractions de 50 cm³. Les fractions contenant le produit attendu sont concentrées sous pression réduite (2,7 kPa) à une température voisine de 40°C pour donner 0,900 g [(3R,4R)-3-hydroxy-5-méthylthio-N-méthyl-L-leucine]¹ [(2R)-(2'-diméthylamino)-éthylthiosarcosine]³ [4'-hydroxy-N-méthyl-L-leucine]⁴ cyclosporine A sous la forme d'un solide blanc fondant à une température voisine de 154°C.

Spectre de R.M.N. ¹H (400 MHz, CDCl₃, δ en ppm) : 1,36 (d, J = 7,5 Hz, 3H : CH₃ 7β) ; 2,04 (s, 3H : CH₃ 1ε) ; 2,25 (s, 6H : N(CH₃)₂ du 2-diméthyl aminoéthylthio en 3α) ; de 2,25 à 2,85 (mt, 7H : les 7H correspondant au CH₂ en 1γ - NCH₂CH₂S du 2-diméthyl aminoéthylthio en 3α et 1H du CH₂ en 4β) ; 2,70 - 2,72 - 3,13 - 3,18 - 3,28 - 3,45 et 3,51 (7 s, 3H chacun : les 7 NCH₃) ; 3,64 (d, J = 6,5 Hz, 1H : OH en 1β) ; 3,86 (mt, H : CH 1β) ; 4,53 (mt, 1H : CH 7α) ; 4,67 (t, J = 9 Hz, 1H : CH 5α) ; 4,83 (mt, 1H : CH 8α) ; de 4,95 à 5,15 (mt, 3H : CH 2α et CH α de deux leucines) ; 5,15 (d, J = 11 Hz, 1H : CH 11α) ; 5,42 (d, J = 6,5 Hz, 1H : CH 1α) ; 5,46 (t, J = 6 Hz, 1H : CH 4α) ; 5,71 (dd, J = 11 et 4 Hz, 1H : CH a d'une leucine) ; 6,00 (s, 1H : CH 3α) ; 7,22 (d, J = 8 Hz, 1H : CONH en 8) ; 7,50 (d, J = 9 Hz, 1H : CONH en 5) ; 7,72 (d, J = 7,5 Hz, 1H : CONH en 7) ; 8,03 (d, J = 10 Hz, 1H : CONH en 2).

La [(3R,4R)-3-hydroxy-5-méthylthio-N-méthyl-L-leucine]¹[4'-hydroxy-N-méthyl-L-leucine]⁴ cyclosporine A est préparée selon la méthode suivante :

A une solution de 0,6 g de [(3R,4R)-3-acétyloxy-5-méthylthio-N-méthyl-L-leucine]¹[4'-acétyloxy-N-méthyl-L-leucine]⁴ cyclosporine A dans 25 cm³ de méthanol, on ajoute en 20 minutes 2 cm³ d'une solution de méthylate de sodium 0,5 M. Le mélange réactionnel est agité à une température voisine de 20°C durant 60 heures. Le mélange résultant est alors concentré sous pression réduite (2,7 kPa) à une température voisine de 30°C. Le résidu est repris par 50 cm³ d'eau distillée. Le solide formé est filtré est rincé par 150 cm³ au total d'eau distillée. Le solide est séché à poids constant puis purifié par chromatographie sur une colonne de silice (0,020 - 0,045 mm) éluée par un mélange d'acétate d'éthyle et de méthanol 9:1 (en volumes) en recueillant des fractions de 50 cm³. Les fractions contenant le produit attendu (fractions 9 à 12) sont concentrées sous pression réduite (2,7 kPa) à une température voisine de 40°C pour donner 0,20 g de [(3R,4R)-3-hydroxy-5-méthylthio-N-méthyl-L-leucine]¹[4'-hydroxy-N-méthyl-L-leucine]⁴ cyclosporine A sous la forme d'une meringue blanche amorphe

Spectre de R.M.N. ¹H (400 MHz, CDCl₃, δ en ppm) : 1,36 (d, J = 7,5 Hz, 3H : CH₃ 7β) ; 1,85 (s, 3H : CH₃ 1ε) ; 2,35 et 2,83 (2 dd, respectivement J = 13 et 9 Hz et J = 13 et 3,5 Hz, 1H chacun : CH₂ 1δ) ; 2,42 (mt, 1H : CH 5β) ; 2,70 - 2,72 - 3,14 - 3,16 - 3,26 - 3,40 et 3,51 (7 s, 3H chacun : les 7 NCH₃) ; 3,18 et 4,69 (2 d, J = 14 Hz, 1H chacun : CH₂ 3α) ; de 3,80 à 3,95 (mt, 2H : CH 1β et OH en 1β) ; 4,52 (mt, 1H : CH 7α) ; 4,67 (t, J = 9 Hz, 1H : CH 5α) ; 4,84 (mt, 1H : CH 8α) ; de 4,95 à 5,10 (mt, 3H : CH 2α et CH α de deux leucines) ; 5,15 (d, J = 11 Hz, 1H : CH 11α) ; 5,42 (d, J = 6,5 Hz, 1H : CH 1α) ; 5,57 (t, J = 5,5 Hz, 1H : CH 4α) ; 5,71 (dd, J = 11 et 4 Hz, 1H : CH α d'une leucine) ; 7,21 (d, J = 8 Hz, 1H : CONH en 8) ; 7,68 (d, J = 9 Hz, 1H : CONH en 5) ; 7,73 (d, J = 7,5 Hz, 1H : CONH en 7) ; 8,05 (d, J = 10 Hz, 1H : CONH en 2).

La [(3R,4R)-3-acétyloxy-5-méthylthio-N-méthyl-L-leucine]¹[4'-acétyloxy- N-méthyl-L-leucine]⁴ cyclosporine A est préparée selon la méthode suivante:

A une solution de 6 g de [(3R,4R)-3-acétyloxy-5-carboxy-N-méthyl-L-leucine]¹[4'-acétyloxy-N-méthyl-L-leucine]⁴ cyclosporine A dans 60 cm³ de 1,2-dichloroéthane refroidie à approximativement -15°C sous atmosphère inerte et à l'abri de la lumière, on ajoute successivement 0,56 cm³ de N-méthylmorpholine (préalablement distillée) et 0,66 cm³ de chloroformiate d'isobutyle (préalablement distillé). Le mélange réactionnel est agité à une température voisine de -15°C durant 150 minutes. On ajoute ensuite en approximativement 15 minutes, une solution de 0,700 g de N-oxyde-2-mercaptopyridine et de 0,78 cm³ de triéthylamine dans 15 cm³ 1,2-dichloréthane. Le mélange réactionnel est agité durant 17 heures à une température voisine de -20°C avant d'ajouter en 10 minutes, 4,09 cm³ de disulfure de diméthyle (préalablement distillé). Le mélange réactionnel est irradié durant 3 heures à l'aide de deux lampes au tungstène de 60W en maintenant la température entre 0 et 10°C. Le mélange résultant est alors concentré sous pression réduite (2,7 kPa) à une température voisine de 40°C. Le résidu est repris par 200 cm³ d'éther de diéthyle et 20 cm³ d'une solution 1 N d'acide chlorhydrique. La phase organique est décantée, lavée successivement avec 150 cm3 au total d'eau distillée, 50 cm³ d'une solution aqueuse molaire d'éthanolamine et 150 cm³ d'eau distillée. La phase organique est séchée sur sulfate de magnésium, filtrée puis concentrée sous pression réduite (2,7 kPa) à une température voisine de 40°C. Le solide résiduel (4 g) est purifié par chromatographie sur une colonne de silice (0,020 - 0,045 mm) éluée par un mélange d'acétate d'éthyle et de méthanol 19:1 (en volumes) en recueillant des fractions de 50 cm³. Les fractions contenant le produit attendu (fractions 30 à 35) sont concentrées sous pression réduite (2,7 kPa) à une température voisine de 40°C pour donner 1,6 g de [(3R,4R)-3-acétyloxy-5-méthylthio-N-méthyl-L-leucine]¹[4'-acétyloxy-N-méthyl-L-leucine]⁴ cyclosporine A sous la forme d'une meringue blanche amorphe.

Spectre de R.M.N. ¹H (400 MHz, CDCl₃, δ en ppm): 1,27 (d large, J = 7,5 Hz, 3H : CH₃ 8β) ; 1,32 (d large, J = 7,5 Hz, 3H : CH₃ 7β) ; 1,45 et 1,50 (2s, 3H chacun : les deux CH₃ en 4γ) ; 1 , 91 (s, 6H : OCOCH₃ en 4γ et CH₃ 1ε) ; 2,01 (s, 3H : OCOCH₃ en 1β) ; 2,30 et 2,54 (respectivement dd et d large, J = 16 et 10 Hz et J = 16 Hz, 1H chacun : CH₂ 4β) ; de 2,35 à 2,50 (mt, 1H : CH 5β) ; 2, 66 - 2,68 - 3,10 - 3,21 - 3,27 - 3,30 et 3,46 (7 s, 3H chacun : 7 NCH₃) ; de 3,00 à 3,15 et 4,62 (respectivement mt et d, J = 14 Hz, 1H chacun : CH₂ 3α) ; 4,42 (mt, 1H : CH 7α) ; 4,77 (t, J = 9 Hz, 1H : CH 5α) ; 4,86 (mt, 1H : CH 8α) ; de 4,90 à 5,00 (mt, 1H : CH 2α) ; 4,99 (d, J = 11 Hz, 1H : CH 11α) ; 5,12 (mt, 1H : CH α d'une leucine) ; 5,35 (d large, J = 12 Hz, 1H : CH α d'une leucine) ; 5,44 (d large, J = 12 Hz, 1H : CH 1α) ; 5,53 (d large, J = 12 Hz, 1H : CH 1β) ; de 5,60 à 5,75 (mt, 2H : CH α d'une leucine et CH 4α) ; 7,50 (d, J = 8 Hz, 1H : CONH en 8) ; 7,63 (d, J = 9 Hz, 1H : CONH en 5) ; 8,08 (d, J = 6,5 Hz, 1H : CONH en 7) ; 8,51 (d, J = 9,5 Hz, 1H : CONH en 2).

La [(3R,4R)-3-acétyloxy-5-carboxy-N-méthyl-L-leucine]¹[4'-acétyloxy-N-méthyl-L-leucine]⁴ cyclosporine A est préparée selon la méthode suivante:

A une solution de 12 g de {[(4R)-4-butèn-2-yl-(E)]-O-acetyl-N,4-dimethylthréonine}¹[4'-acétyloxy- N-méthyl-L-leucine]⁴ cyclosporine A dans 750 cm³ de t-butanol, on ajoute successivement en agitant vigoureusement, une solution de 7,6 g de carbonate de sodium dans 200 cm³ d'eau distillée puis uns solution de 15,7 metaperiodate de sodium dans 200 cm³ d'eau distillée. Une solution de 0,29 g de permanganate de potassium dans 200 cm³ d'eau distillée est ajoutée goutte-à-goutte en approximativement une heure en maintenant le mélange réactionnel à une température inférieure à 30°C. Le mélange réactionnel est agité durant 36 h à une température voisine de 20°C puis une solution de 0,10 g de permanganate de potassium dans 70 cm³ d'eau distillée est ajoutée goutte-à-goutte en approximativement 30 minutes. Le mélange réactionnel est agité à une température voisine de 20°C durant 3 heures puis une solution de 47 g de métabisulfite de potassium dans 200 cm³ d'eau distillée est ajoutée goutte-à-goutte en approximativement 30 minutes. Après addition, le mélange réactionnel est agité durant 15 minutes puis 200 cm³ d'une solution 2N d'acide sulfurique est ajoutée goutte-à-goutte en en approximativement 10 minutes. Le mélange réactionnel est extrait par 900 cm³ au total de diéthyléther. Les phases organiques réunies sont séchées sur sulfate de magnésium, puis concentrées sous pression réduite (2,7 kPa), à une température voisine de 40°C. Le solide résiduel (11,18 g) est agite durant 30 minutes avec 300 cm³ d'eau distillée. Le solide obtenu est filtré, rincé avec 200 cm³ au total d'eau distillée puis en suspension dans 200 cm³ d'eau distillée. A la suspension obtenue, on ajoute 1,1 cm³ d'éthanolamine. Le mélange réactionnel est agité durant 90 minutes à une température voisine de 20°C. L'insoluble est filtré, le filtrat est extrait par 400 cm³ au total de diéthyléther. La phase aqueuse est acidifiée à pH =1 avec une solution 5N d'acide chlorhydrique. Le mélange réactionnel est extrait par 700 cm³ au total d'acétate d'éthyle. Les phases organiques réunies sont lavée par 200 cm³ au total d'eau distillée, séchées sur sulfate de magnésium puis concentrées sous pression réduite (2,7 kPa) à une température voisine de 40°C pour donner 6,78 g de [(3R,4R)-3-acétyloxy-5-carboxy-N-méthyl-L-leucine]¹[4'-acétyloxy- N-méthyl-L-leucine]⁴ cyclosporine A sous la forme d'une meringue blanche amorphe.

Spectre de R.M.N. ¹H (400 MHz, CDCl₃, δ en ppm) : 1,91 (s, 3H : OCOCH₃ en 4γ) ; 2,02 (s, 3H : OCOCH₃ en 1β) ; 2,25 et 2,50 (2 dd, respectivement J = 16 et 10 Hz et J = 16 et 3 Hz, 1H chacun : CH₂ en 4β) ; 2,37 (mt, 1H : CH 5β) ; 2,67 - 2,69 - 3,11 - 3,22 - 3,25 et 3,47 (6s, respectivement 3H - 3H - 3H - 6H - 3H et 3H : les 7 NCH₃) ; 4,45 (mt, 1H : CH 7α) ; 4,60 (d, J = 14 Hz, 1H : 1H du CH₂ 3α) ; 4,80 (mt, 1H : CH 5α) ; 4,85 (mt, 1H : CH 8α) ; de 4,95 à 5,05 (mt, 1H : CH 2α) ; 5,00 (d, J = 11 Hz, 1H : CH 11α) ; 5,15 (mt, 1H : CH α d'une leucine) ; de 5,30 à 5,65 (mt, 4H : CH α d'une leucine - CH 4α - CH 1α et CH 1β) ; 5,68 (dd, J = 11 et 4 Hz, 1H : CH α d'une leucine) ; 7,40 (d, J = 8 Hz, 1H : CONH en 8) ; 7,71 (d, J = 9 Hz, 1H : CONH en 5) ; 7,95 (d, J = 7,5 Hz, 1H : CONH en 7) ; 8,31 (d, J = 9,5 Hz, 1H : CONH en 2).

La {[(4R)-4-butèn-2-yl-(E)]-O-acetyl-N,4-dimethylthréonine}¹[4'-acétyloxy- N-méthyl-L-leucine]⁴ cyclosporine A est préparée selon la méthode suivante:

A une solution de 17,95 g de [4'-hydroxy-N-méthyl-L-leucine]⁴ cyclosporine A dans 295 cm³ de dichlorométhane, on ajoute successivement 10,77 g de 4-diméthylaminopyridine, 12,40 cm³ de triéthylamine et 5,6 cm³ d'anhydride acétique. Le mélange est agité durant 36 h à une température voisine de 20°C puis 30 cm³ d'eau distillée sont ajoutés en approximativement 45 minutes. La phase organique est décantée puis lavée par 180 cm³ au total d'eau distillée, séchée sur sulfate de magnésium, puis concentrées sous pression réduite (2,7 kPa), à une température voisine de 40°C. Le solide résiduel (24,07 g) est agite durant 12 h avec 150 cm³ d'eau distillée. Le solide obtenu est filtré, rincé avec 100 cm³ au total d'eau distillée puis séché sous pression atmosphérique à une température voisine de 20°C. Le solide résiduel (21,45 g) est purifié par chromatographie sur une colonne de silice (0,020 - 0,045 mm) éluée par un mélange d'acétate d'éthyle et de méthanol 19:1 (en volumes) en recueillant des fractions de 50 cm³. Les fractions contenant le produit attendu (fractions 7 à 15) sont concentrées sous pression réduite (2,7 kPa) à une température voisine de 40°C pour donner 10,5 g de {[(4R)-4-butèn-2-yl-(E)]-O-acetyl-N,4-dimethylthréonine}¹[4'-acétyloxy- N-méthyl-L-leucine]⁴ cyclosporine A sous la forme d'une meringue blanche amorphe fondant à une température voisine de 164°C.

Spectre de R.M.N. ¹H (400 MHz, CDCl₃, δ en ppm) : 1,27 (d, J = 7 Hz, 3H : CH₃ 8β) ; 1,33 (d, J = 7,5 Hz, 3H : CH₃ 7β) ; 1,45 et 1,50 (2 s, 3H chacun : les deux CH₃ en 4γ) ; 1,59 (d, J = 5 Hz, 3H : CH₃ 1η) ; 1,90 (s, 3H : OCOCH₃ en 4γ) ; 2,01 (s, 3H : OCOCH₃ en 1β) ; 2,31 et 2,55 (2dd, respectivement J = 16 et 11 Hz et J = 16 et 2 Hz, 1H chacun : CH₂ 4β) ; 2,43 (mt, 1H : CH 5β) ; 2,65 - 2,68 - 3,13 - 3,20 - 3,23 - 3,25 et 3,47 (7 s, 3H chacun : les 7 NCH₃) ; 3,08 et 4,62 (2 d, J = 14 Hz, 1H chacun : CH₂ 3α) ; 4,42 (mt, 1H : CH 7α) ; 4,78 (t, J = 9 Hz, 1H : CH 5α) ; 4,85 (mt, 1H : CH 8α) ; 4,45 (mt, 1H : CH 2α) ; 4,98 (d, J = 11 Hz, 1H : CH 11α) ; de 5,10 à 5,20 (mt, 2H : CH α de deux leucines) ; de 5,20 à 5,35 (mt, 2H : CH=CH) ; 5,52 (s, 2H : CH 1α et CH 1β) ; 5,58 (dd, J = 11 et 2 Hz, 1H : CH 4α) ; 5,68 (dd, J = 11 et 4 Hz, 1H : CH α d'une leucine) ; 7,42 (d, J = 8 Hz, 1H : CONH en 8) ; 7,57 (d, J = 9 Hz, 1H : CONH en 5) ; 8,04 (d, J = 7 Hz, 1H : CONH en 7) ; 8,51 (d, J = 10 Hz, 1H : CONH en 2).

La [4'-hydroxy-N-méthyl-L-leucine]⁴ cyclosporine A peut être préparé selon la méthode décrite dans le brevet EP484281

### Exemple 7

En opérant de manière analogue aux méthodes décrites dans les exemples précédents, les produits suivants sont préparés :
[(3R,4R)-3-hydroxy-5-méthylthio-N-méthyl-L-leucine]¹[(R)-2-aminoéthylthio-Sar]³-cyclosporine A ;
[(3R,4R)-3-hydroxy-5-méthylthio-N-méthyl-L-leucine]¹[(R)-2-(N-méthylamino)éthylthio-Sar]³-cyclosporine A ;
[(3R,4R)-3-hydroxy-5-méthylthio-N-méthyl-L-leucine]¹[(R)-2-(N-éthylamino)éthylthio-Sar]³-cyclosporine A ;
[(3R,4R)-3-hydroxy-5-méthylthio-N-méthyl-L-leucine]¹[(R)-2-(N-iso-propylamino)éthylthio-Sar]³-cyclosporine A ;
[(3R,4R)-3-hydroxy-5-méthylthio-N-méthyl-L-leucine]¹[(R)-2-(N-tert-butylamino)éthylthio-Sar]³-cyclosporine A ;
[(3R,4R)-3-hydroxy-5-méthylthio-N-méthyl-L-leucine]¹[(R)-2-(N-phénylamino)éthylthio-Sar]³-cyclosporine A ;
[(3R,4R)-3-hydroxy-5-méthylthio-N-méthyl-L-leucine]¹[(R)-2-(N-benzylamino)éthylthio-Sar]³-cyclosporine A ;
[(3R,4R)-3-hydroxy-5-méthylthio-N-méthyl-L-leucine]¹[(R)-2-(N-méthyl-N-éthylamino)éthylthio-Sar]³-cyclosporine A ;
[(3R,4R)-3-hydroxy-5-méthylthio-N-méthyl-L-leucine]¹[(R)-2-(N-méthyl-N-iso-propylamino)éthylthio-Sar]³-cyclosporine A ;
[(3R,4R)-3-hydroxy-5-méthylthio-N-méthyl-L-leucine]¹[(R)-2-(N-méthyl-N-tert-butylamino)éthylthio-Sar]³-cyclosporine A ;
[(3R,4R)-3-hydroxy-5-méthylthio-N-méthyl-L-leucine]¹[(R)-2-(N-méthyl-N-allylamino)éthylthio-Sar]³-cyclosporine A ;
[(3R,4R)-3-hydroxy-5-méthylthio-N-méthyl-L-leucine]¹[(R)-2-(N-méthyl-N-phénylamino)éthylthio-Sar]³-cyclosporine A ;
[(3R,4R)-3-hydroxy-5-méthylthio-N-méthyl-L-leucine]¹[(R)-2-(N-méthyl-N-benzylamino)éthylthio-Sar]³-cyclosporine A ;
[(3R,4R)-3-hydroxy-5-méthylthio-N-méthyl-L-leucine]¹[(R)-2-(N,N-diéthylamino)éthylthio-Sar]³-cyclosporine A ;
[(3R,4R)-3-hydroxy-5-méthylthio-N-méthyl-L-leucine]¹[(R)-2-(N,N-di-iso-propylamino)éthylthio-Sar]³-cyclosporine A ;
[(3R,4R)-3-hydroxy-5-méthylthio-N-méthyl-L-leucine]¹[(R)-2-(N,N-diallylamino)éthylthio-Sar]³-cyclosporine A ;
[(3R,4R)-3-hydroxy-5-méthylthio-N-méthyl-L-leucine]¹[(R)-2-(1-pipéridyl)éthylthio-Sar]³-cyclosporine A ;
[(3R,4R)-3-hydroxy-5-méthylthio-N-méthyl-L-leucine]¹[(R)-3-aminopropylthio-Sar]³-cyclosporine A ;
[(3R,4R)-3-hydroxy-5-méthylthio-N-méthyl-L-leucine]¹[(R)-3-(N-méthylamino)propylthio-Sar]³-cyclosporine A ;
[(3R,4R)-3-hydroxy-5-méthylthio-N-méthyl-L-leucine]¹[(R)-3-(N-éthylamino)propylthio-Sar]³-cyclosporine A ;
[(3R,4R)-3-hydroxy-5-méthylthio-N-méthyl-L-leucine]¹[(R)-3-(N-iso-propylamino)propylthio-Sar]³-cyclosporine A ;
[(3R,4R)-3-hydroxy-5-méthylthio-N-méthyl-L-leucine]¹[(R)-3-(N-tert-butylamino)propylthio-Sar]³-cyclosporine A ;
[(3R,4R)-3-hydroxy-5-méthylthio-N-méthyl-L-leucine]¹[(R)-3-(N-phénylamino)propylthio-Sar]³-cyclosporine A ;
[(3R,4R)-3-hydroxy-5-méthylthio-N-méthyl-L-leucine]¹[(R) -3- (N-benzylamino)propylthio-Sar]³-cyclosporine A ;
[(3R,4R)-3-hydroxy-5-méthylthio-N-méthyl-L-leucine]¹[(R)-3-(N-méthyl-N-éthylamino)propylthio-Sar]³-cyclosporine A ;
[(3R,4R)-3-hydroxy-5-méthylthio-N-méthyl-L-leucine]¹[(R)-3-(N-méthyl-N-iso-propylamino)propylthio-Sar]³-cyclosporine A ;
[(3R,4R)-3-hydroxy-5-méthylthio-N-méthyl-L-leucine]¹[(R)-3-(N-méthyl-N-tert-butylamino)propylthio-Sar]³-cyclosporine A ;
[(3R,4R)-3-hydroxy-5-méthylthio-N-méthyl-L-leucine]¹[(R)-3-(N-méthyl-N-allylamino)propylthio-Sar]³-cyclosporine A ;
[(3R,4R)-3-hydroxy-5-méthylthio-N-méthyl-L-leucine]¹[(R)-3-(N-méthyl-N-phénylamino)propylthio-Sar]³-cyclosporine A ;
[(3R,4R)-3-hydroxy-5-méthylthio-N-méthyl-L-leucine]¹[(R)-3-(N-méthyl-N-benzylamino)propylthio-Sar]³-cyclosporine A ;
[(3R,4R)-3-hydroxy-5-méthylthio-N-méthyl-L-leucine]¹[(R)-2-(N,N-diméthylamino)éthylthio-Sar]³-cyclosporine A ;
[(3R,4R)-3-hydroxy-5-méthylthio-N-méthyl-L-leucine]¹[(R)-3-(N,N-diéthylamino)propylthio-Sar]³-cyclosporine A ;
[(3R,4R)-3-hydroxy-5-méthylthio-N-méthyl-L-leucine]¹[(R)-3-(N,N-di-iso-propylamino)propylthio-Sar]³-cyclosporine A ;
[(3R,4R)-3-hydroxy-5-méthylthio-N-méthyl-L-leucine]¹[(R)-3-(N,N-diallyalamino)propylthio-Sar]³-cyclosporine A ;
[(3R,4R)-3-hydroxy-5-méthylthio-N-méthyl-L-leucine]¹[(R)-3-(1-pipéridyl)propylthio-Sar]³⁻cyclosporine A ;
[(3R,4R)-3-hydroxy-5-méthylthio-N-méthyl-L-leucine]¹[(R)-4-aminobutylthio-Sar]³-cyclosporine A ;
[(3R,4R)-3-hydroxy-5-méthylthio-N-méthyl-L-leucine]¹[(R)-4-(N-méthylamino)butylthio-Sar]³-cyclosporine A ;
[(3R,4R)-3-hydroxy-5-méthylthio-N-méthyl-L-leucine]¹[(R)-4-(N-éthylamino)butylthio-Sar]³-cyclosporine A ;
[(3R,4R)-3-hydroxy-5-méthylthio-N-méthyl-L-leucine]¹[(R)-4-(N-iso-propylamino)butylthio-Sar]³-cyclosporine A ;
[(3R,4R)-3-hydroxy-5-méthylthio-N-méthyl-L-leucine]¹[(R)-4-(N-tert-butylamino)butylthio-Sar]³-cyclosporine A ;
[(3R,4R)-3-hydroxy-5-méthylthio-N-méthyl-L-leucine]¹[(R)-4-(N-phénylamino)butylthio-Sar]³-cyclosporine A ;
[(3R,4R)-3-hydroxy-5-méthylthio-N-méthyl-L-leucine]¹[(R)-4-(N-benzylamino)butylthio-Sar]³-cyclosporine A ;
[(3R,4R)-3-hydroxy-5-méthylthio-N-méthyl-L-leucine]¹[(R)-4-(N-méthyl-N-éthylamino)butylthio-Sar]³-cyclosporine A ;
[(3R,4R)-3-hydroxy-5-méthylthio-N-méthyl-L-leucine]¹[(R)-4-(N-méthyl-N-iso-propylamino)butylthio-Sar]³-cyclosporine A ;
[(3R,4R)-3-hydroxy-5-méthylthio-N-méthyl-L-leucine]¹[(R)-4-(N-méthyl-N-tert-butylamino)butylthio-Sar]³-cyclosporine A ;
[(3R,4R)-3-hydroxy-5-méthylthio-N-méthyl-L-leucine]¹[(R)-4-(N-méthyl-N-allylamino)butylthio-Sar]³-cyclosporine A ;
[(3R,4R)-3-hydroxy-5-méthylthio-N-méthyl-L-leucine]¹[(R)-4-(N-méthyl-N-phénylamino)butylthio-Sar]³-cyclosporine A ;
[(3R,4R)-3-hydroxy-5-méthylthio-N-méthyl-L-leucine]¹[(R)-4-(N-méthyl-N-benzylamino)butylthio-Sar]³-cyclosporine A ;
[(3R,4R)-3-hydroxy-5-méthylthio-N-méthyl-L-leucine]¹[(R)-4-(N,N-diméthylamino)butylthio-Sar]³-cyclosporine A ;
[(3R,4R)-3-hydroxy-5-méthylthio-N-méthyl-L-leucine]¹[(R)-4-(N,N-diéthylamino)butylthio-Sar]³-cyclosporine A ;
[(3R,4R)-3-hydroxy-5-méthylthio-N-méthyl-L-leucine]¹[(R)-4-(N,N-di-iso-propylamino)butylthio-Sar]³-cyclosporine A ;
[(3R,4R)-3-hydroxy-5-méthylthio-N-méthyl-L-leucine]¹[(R)-4-(N,N-diallyalamino)butylthio-Sar]³-cyclosporine A ;
[(3R,4R)-3-hydroxy-5-méthylthio-N-méthyl-L-leucine]¹[(R)-4-(1-pipéridyl)butylthio-Sar]³-cyclosporine A ;
[(3R,4R)-3-hydroxy-5-méthylthio-N-méthyl-L-leucine]¹[(R)-2-amino-2-méthylpropylthio-Sar]³-cyclosporine A ;
[(3R,4R)-3-hydroxy-5-méthylthio-N-méthyl-L-leucine]¹[(R)-2-(N,N-diméthylamino)-2-méthylpropylthio-Sar]³-cyclosporine A ;
[(3R,4R)-3-hydroxy-5-méthylthio-N-méthyl-L-leucine]¹[(R)-2-(N,N-diéthylamino)-2-méthylpropylthio-Sar]³-cyclosporine A ;
[(3R,4R)-3-hydroxy-5-méthylthio-N-méthyl-L-leucine]¹[(R)-2-(1-pipéridyl)-2-méthylpropylthio-Sar]³-cyclosporine A ;
[(3R,4R)-3-hydroxy-5-méthylthio-N-méthyl-L-leucine]¹[(R)- 3-amino-3-méthylbutylthio-Sar]³-cyclosporine A ;
[(3R,4R)-3-hydroxy-5-méthylthio-N-méthyl-L-leucine]¹[(R)-3-(N,N-diméthylamino)-3-méthylbutylthio-Sar]³-cyclosporine A ;
[(3R,4R)-3-hydroxy-5-méthylthio-N-méthyl-L-leucine]¹[(R)-3-(N,N-diéthylamino)-3-méthylbutylthio-Sar]³-cyclosporine A ;
[(3R,4R)-3-hydroxy-5-méthylthio-N-méthyl-L-leucine]¹[(R)-3-(1-pipéridyl)-3-méthylbutylthio-Sar]³-cyclosporine A
[(3R,4R)-3-hydroxy-5-méthylthio-N-méthyl-L-leucine]¹[(R)-2-(1-morpholino)éthylthio-Sar]³-cyclosporine A ;
[(3R,4R)-3-hydroxy-5-méthylthio-N-méthyl-L-leucine]¹[(R)-2-(1-azétidino)éthylthio-Sar]³-cyclosporine A ;
[(3R,4R)-3-hydroxy-5-méthylthio-N-méthyl-L-leucine]¹{(R)-2-[1-(4-méthylpipérazino)]éthylthio-Sar}³-cyclosporine A ;
[(3R,4R)-3-hydroxy-5-méthylthio-N-méthyl-L-leucine]¹{(R)-2-[1-(4-phénylpipérazino)]éthylthio-Sar}³-cyclosporine A ;
[(3R,4R)-3-hydroxy-5-méthylthio-N-méthyl-L-leucine]¹{(R)-2-[1-(4-benzylpipérazino)]éthylthio-Sar}³-cyclosporine A ;
[(3R,4R)-3-hydroxy-5-méthylthio-N-méthyl-L-leucine]¹{(R)-2-[1-(4-méthyl-1,2,3,6-tétrahydropyridyl)]éthylthio-Sar}³-cyclosporine A ;
[(3R,4R)-3-hydroxy-5-méthylthio-N-méthyl-L-leucine]¹{(R)-2-[1-(4-phényl-1,2,3,6-tétrahydropyridyl)]éthylthio-Sar}³-cyclosporine A ;
[(3R,4R)-3-hydroxy-5-méthylthio-N-méthyl-L-leucine]¹[(R)-3-(1-morpholino)propylthio-Sar]³-cyclosporine A ;
[(3R,4R)-3-hydroxy-5-méthylthio-N-méthyl-L-leucine]¹[(R)-3-(1-azétidino)propylthio-Sar]³-cyclosporine A ;
[(3R,4R)-3-hydroxy-5-méthylthio-N-méthyl-L-leucine]¹{(R)-3-[1-(4-méthylpipérazino)]propylthio-Sar}³-cyclosporine A ;
[(3R,4R)-3-hydroxy-5-méthylthio-N-méthyl-L-leucine]¹{(R)-3-[1-(4-phénylpipérazino)]propylthio-Sar}³-cyclosporine A ;
[(3R,4R)-3-hydroxy-5-méthylthio-N-méthyl-L-leucine]¹{(R)-3-[1-(4-benzylpipérazino)]propylthio-Sar}³-cyclosporine A ;
[(3R,4R)-3-hydroxy-5-méthylthio-N-méthyl-L-leucine]¹{(R)-3-[1-(4-méthyl-1,2,3,6-tétrahydropyridyl)]propylthio-Sar}³-cyclosporine A ;
[(3R,4R)-3-hydroxy-5-méthylthio-N-méthyl-L-leucine]¹{(R)-3-[1-(4-méthyl-1,2,3,6-tétrahydropyridyl)}propylthio-Sar}³-cyclosporine A.
[(3R,4R)-3-hydroxy-5-méthylthio-N-méthyl-L-leucine]¹[(R)-2-méthylthio-Sar]³-[4'-hydroxy-MeLeu]⁴-cyclosporine A ;
[(3R,4R)-3-hydroxy-5-méthylthio-N-méthyl-L-leucine]¹[(R)-2-aminoéthylthio-Sar]³-[4'-hydroxy-MeLeu]⁴-cyclosporine A ;
[(3R,4R)-3-hydroxy-5-méthylthio-N-méthyl-L-leucine]¹[(R)-2-(N-méthylamino)éthylthio-Sar]³-[4'-hydroxy-MeLeu]⁴-cyclosporine A ;
[(3R,4R)-3-hydroxy-5-méthylthio-N-méthyl-L-leucine]¹[(R)-2-(N-éthylamino)éthylthio-Sar]³-[4'-hydroxy-MeLeu]⁴-cyclosporine A ;
[(3R,4R)-3-hydroxy-5-méthylthio-N-méthyl-L-leucine]¹[(R)-2-(N-iso-propylamino)éthylthio-Sar]³-[4'-hydroxy-MeLeu]⁴-cyclosporine A ;
[(3R,4R)-3-hydroxy-5-méthylthio-N-méthyl-L-leucine]¹[(R)-2-(N-tert-butylamino)éthylthio-Sar]³-[4'-hydroxy-MeLeu]⁴-cyclasporine A ;
[(3R,4R)-3-hydroxy-5-méthylthio-N-méthyl-L-leucine]¹[(R)-2-(N-phénylamino)éthylthio-Sar]³-[4'-hydroxy-MeLeu]⁴-cyclosporine A ;
[(3R,4R)-3-hydroxy-5-méthylthio-N-méthyl-L-leucine]¹[(R)-2-(N-benzylamino)éthylthio-Sar]³-[4'-hydroxy-MeLeu]⁴-cyclosporine A ;
[(3R,4R)-3-hydroxy-5-méthylthio-N-méthyl-L-leucine]¹[(R)-2-(N-méthyl-N-éthylamino)éthylthio-Sar]³-[4'-hydroxy-MeLeu]⁴-cyclosporine A ;
[(3R,4R)-3-hydroxy-5-méthylthio-N-méthyl-L-leucine]¹[(R)-2-(N-méthyl-N-iso-propylamino)éthylthio-Sar]³-[4'-hydroxy-MeLeu]⁴-cyclosporine A ;
[(3R,4R)-3-hydroxy-5-méthylthio-N-méthyl-L-leucine]¹[(R)-2-(N-méthyl-N-tert-butylamino)éthylthio-Sar]³-[4'-hydroxy-MeLeu]⁴-cyclosporine A ;
[(3R,4R)-3-hydroxy-5-méthylthio-N-méthyl-L-leucine]¹[(R)-2-(N-méthyl-N-allylamino)éthylthio-Sar]³-[4'-hydroxy-MeLeu]⁴-cyclosporine A ;
[(3R,4R)-3-hydroxy-5-méthylthio-N-méthyl-L-leucine]¹[(R)-2-(N-méthyl-N-phénylamino)éthylthio-Sar]³-[4'-hydroxy-MeLeu]⁴-cyclosporine A ;
[(3R,4R)-3-hydroxy-5-méthylthio-N-méthyl-L-leucine]¹[(R)-2-(N-méthyl-N-benzylamino)éthylthio-Sar]³-[4'-hydroxy-MeLeu]⁴-cyclosporine A ;
[(3R,4R)-3-hydroxy-5-méthylthio-N-méthyl-L-leucine]¹[(R)-2-(N,N-diéthylamino)éthylthio-Sar]³-[4'-hydroxy-MeLeu]⁴-cyclosporine A ;
[(3R,4R)-3-hydroxy-5-méthylthio-N-méthyl-L-leucine]¹[(R)-2-(N,N-di-iso-propylamino)éthylthio-Sar]³-[4'-hydroxy-MeLeu]⁴-cyclosporine A ;
[(3R,4R)-3-hydroxy-5-méthylthio-N-méthyl-L-leucine]¹[(R)-2-(N,N-diallylamino)éthylthio-Sar]³-[4'-hydroxy-MeLeu]⁴-cyclosporine A ;
[(3R,4R)-3-hydroxy-5-méthylthio-N-méthyl-L-leucine]¹[(R)-2-(1-pipéridyl)éthylthio-Sar]³-[4'-hydroxy-MeLeu]⁴-cyclosporine A ;
[(3R,4R)-3-hydroxy-5-méthylthio-N-méthyl-L-leucine]¹[(R)-3-aminopropylthio-Sar]³-[4'-hydroxy-MeLeu]⁴-cyclosporine A ;
[(3R,4R)-3-hydroxy-5-méthylthio-N-méthyl-L-leucine]¹[(R)-3-(N-méthylamino)propylthio-Sar]³-[4'-hydroxy-MeLeu]⁴-cyclosporine A ;
[(3R,4R)-3-hydroxy-5-méthylthio-N-méthyl-L-leucine]¹[(R)-3-(N-éthylamino)propylthio-Sar]³-[4'-hydroxy-MeLeu]⁴-cyclosporine A ;
[(3R,4R)-3-hydroxy-5-méthylthio-N-méthyl-L-leucine]¹[(R)-3-(N-iso-propylamino)propylthio-Sar]³-[4'-hydroxy-MeLeu]⁴-cyclosporine A ;
[(3R,4R)-3-hydroxy-5-méthylthio-N-méthyl-L-leucine]¹[(R)-3-(N-tert-butylamino)propylthio-Sar]³-[4'-hydroxy-MeLeu]⁴-cyclosporine A ;
[(3R,4R)-3-hydroxy-5-méthylthio-N-méthyl-L-leucine]¹[(R)-3-(N-phénylamino)propylthio-Sar]³-[4'-hydroxy-MeLeu]⁴-cyclosporine A ;
[(3R,4R)-3-hydroxy-5-méthylthio-N-méthyl-L-leucine]¹[(R)-3-(N-benzylamino)propylthio-Sar]³-[4'-hydroxy-MeLeu]⁴-cyclosporine A ;
[(3R,4R)-3-hydroxy-5-méthylthio-N-méthyl-L-leucine]¹[(R)-3-(N-méthyl-N-éthylamino)propylthio-Sar]³-[4'-hydroxy-MeLeu]⁴-cyclosporine A ;
[(3R,4R)-3-hydroxy-5-méthylthio-N-méthyl-L-leucine]¹[(R)-3-(N-méthyl-N-iso-propylamino)propylthio-Sar]³-[4'-hydroxy-MeLeu]⁴-cyclosporine A;
[(3R,4R)-3-hydroxy-5-méthylthio-N-méthyl-L-leucine]¹[(R)-3-(N-méthyl-N-tert-butylamino)propylthio-Sar]³-[4'-hydroxy-MeLeu]⁴-cyclosporine A;
[(3R,4R)-3-hydroxy-5-méthylthio-N-méthyl-L-leucine]¹[(R)-3-(N-méthyl-N-allylamino)propylthio-Sar]³-[4'-hydroxy-MeLeu]⁴-cyclosporine A ;
[(3R,4R)-3-hydroxy-5-méthylthio-N-méthyl-L-leucine]¹[(R)-3-(N-méthyl-N-phénylamino)propylthio-Sar]³-[4'-hydroxy-MeLeu]⁴-cyclosporine A ;
[(3R,4R)-3-hydroxy-5-méthylthio-N-méthyl-L-leucine]¹[(R)-3-(N-méthyl-N-benzylamino)propylthio-Sar]³-[4'-hydroxy-MeLeu]⁴-cyclosporine A ;
[(3R,4R)-3-hydroxy-5-méthylthio-N-méthyl-L-leucine]¹[(R)-2-(N,N-diméthylamino)éthylthio-Sar]³-[4'-hydroxy-MeLeu]⁴-cyclosporine A ;
[(3R,4R)-3-hydroxy-5-méthylthio-N-méthyl-L-leucine]¹[(R)-3-(N,N-diéthylamino)propylthio-Sar]³-[4'-hydroxy-MeLeu]⁴-cyclosporine A ;
[(3R,4R)-3-hydroxy-5-méthylthio-N-méthyl-L-leucine]¹[(R)-3-(N,N-di-iso-propylamino)propylthio-Sar]³-[4'-hydroxy-MeLeu]⁴-cyclosporine A ;
[(3R,4R)-3-hydroxy-5-méthylthio-N-méthyl-L-leucine]¹[(R)-3-(N,N-diallyalamino)propylthio-Sar]³-[4'-hydroxy-MeLeu]⁴-cyclosporine A ;
[(3R,4R)-3-hydroxy-5-méthylthio-N-méthyl-L-leucine]¹[(R)-3-(1-pipéridyl)propylthio-Sar]³-[4'-hydroxy-MeLeu]⁴-cyclosporine A ;
[(3R,4R)-3-hydroxy-5-méthylthio-N-méthyl-L-leucine]¹[(R)-4-aminobutylthio-Sar]³-[4'-hydroxy-MeLeu]⁴-cyclosporine A ;
[(3R,4R)-3-hydroxy-5-méthylthio-N-méthyl-L-leucine]¹[(R)-4-(N-méthylamino)butylthio-Sar]³-[4'-hydroxy-MeLeu]⁴-cyclosporine A ;
[(3R,4R)-3-hydroxy-5-méthylthio-N-méthyl-L-leucine]¹[(R)-4-(N-éthylamino)butylthio-Sar]³-[4'-hydroxy-MeLeu]⁴-cyclosporine A ;
[(3R,4R)-3-hydroxy-5-méthylthio-N-méthyl-L-leucine]¹[(R)-4-(N-iso-propylamino)butylthio-Sar]³-[4'-hydroxy-MeLeu]⁴-cyclosporine A ;
[(3R,4R)-3-hydroxy-5-méthylthio-N-méthyl-L-leucine]¹[(R)-4-(N-tert-butylamino)butylthio-Sar]³-[4'-hydroxy-MeLeu]⁴-cyclosporine A ;
[(3R,4R)-3-hydroxy-5-méthylthio-N-méthyl-L-leucine]¹[(R)-4-(N-phénylamino)butylthio-Sar]³-[4'-hydroxy-MeLeu]⁴-cyclosporine A ;
[(3R,4R)-3-hydroxy-5-méthylthio-N-méthyl-L-leucine]¹[(R)-4-(N-benzylamino)butylthio-Sar]³-[4'-hydroxy-MeLeu]⁴-cyclosporine A ;
[(3R,4R)-3-hydroxy-5-méthylthio-N-méthyl-L-leucine]¹[(R)-4-(N-méthyl-N-éthylamino)butylthio-Sar]³-[4'-hydroxy-MeLeu]⁴-cyclosporine A ;
[(3R,4R)-3-hydroxy-5-méthylthio-N-méthyl-L-leucine]¹[(R)-4-(N-méthyl-N-iso-propylamino)butylthio-Sar]³-[4'-hydroxy-MeLeu]⁴-cyclosporine A;
[(3R,4R)-3-hydroxy-5-méthylthio-N-méthyl-L-leucine]¹[(R)-4-(N-méthyl-N-tert-butylamino)butylthio-Sar]³-[4'-hydroxy-MeLeu]⁴-cyclosporine A;
[(3R,4R)-3-hydroxy-5-méthylthio-N-méthyl-L-leucine]¹[(R)-4-(N-méthyl-N-allylamino)butylthio-Sar]³-[4'-hydroxy-MeLeu]⁴-cyclosporine A ;
[(3R,4R)-3-hydroxy-5-méthylthio-N-méthyl-L-leucine]¹[(R)-4-(N-méthyl-N-phénylamino)butylthio-Sar]³-[4'-hydroxy-MeLeu]⁴-cyclosporine A ;
[(3R,4R)-3-hydroxy-5-méthylthio-N-méthyl-L-leucine]¹[(R)-4-(N-méthyl-N-benzylamino)butylthio-Sar]³-[4'-hydroxy-MeLeu]⁴-cyclosporine A ;
[(3R,4R)-3-hydroxy-5-méthylthio-N-méthyl-L-leucine]¹[(R)-4-(N,N-diméthylamino)butylthio-Sar]³-[4'-hydroxy-MeLeu]⁴-cyclosporine A ;
[(3R,4R)-3-hydroxy-5-méthylthio-N-méthyl-L-leucine]¹[(R)-4-(N,N-diéthylamino)butylthio-Sar]³-[4'-hydroxy-MeLeu]⁴-cyclosporine A ;
[(3R,4R)-3-hydroxy-5-méthylthio-N-méthyl-L-leucine]¹[(R)-4-(N,N-di-iso-propylamino)butylthio-Sar]³-[4'-hydroxy-MeLeu]⁴-cyclosporine A ;
[(3R,4R)-3-hydroxy-5-méthylthio-N-méthyl-L-leucine]¹[(R)-4-(N,N-diallyalamino)butylthio-Sar]³-[4'-hydroxy-MeLeu]⁴-cyclosporine A ;
[(3R,4R)-3-hydroxy-5-méthylthio-N-méthyl-L-leucine]¹[(R)-4-(1-pipéridyl)butylthio-Sar]³-[4'-hydroxy-MeLeu]⁴-cyclosporine A ;
[(3R,4R)-3-hydroxy-5-méthylthio-N-méthyl-L-leucine]¹[(R)-2-amino-2-méthylpropylthio-Sar]³-[4'-hydroxy-MeLeu]⁴-cyclosporine A ;
[(3R,4R)-3-hydroxy-5-méthylthio-N-méthyl-L-leucine]¹[(R)-2-(N,N-diméthylamino)-2-méthylpropylthio-Sar]³-[4'-hydroxy-MeLeu]⁴-cyclosporine A ;
[(3R,4R)-3-hydroxy-5-méthylthio-N-méthyl-L-leucine]¹[(R)-2-(N,N-diéthylamino)-2-méthylpropylthio-Sar]³-[4'-hydroxy-MeLeu]⁴-cyclosporine A ;
[(3R,4R)-3-hydroxy-5-méthylthio-N-méthyl-L-leucine]¹[(R)-2-(1-pipéridyl)-2-méthylpropylthio-Sar]³-[4'-hydroxy-MeLeu]⁴-cyclosporine A ;
[(3R,4R)-3-hydroxy-5-méthylthio-N-méthyl-L-leucine]¹[(R)- 3-amino-3-méthylbutylthio-Sar]³-[4'-hydroxy-MeLeu]⁴-cyclosporine A ;
[(3R,4R)-3-hydroxy-5-méthylthio-N-méthyl-L-leucine]¹[(R)-3-(N,N-diméthylamino)-3-méthylbutylthio-Sar]³-[4'-hydroxy-MeLeu]⁴-cyclosporine A ;
[(3R,4R)-3-hydroxy-5-méthylthio-N-méthyl-L-leucine]¹[(R)-3-(N,N-diéthylamino)-3-méthylbutylthio-Sar]³-[4'-hydroxy-MeLeu]⁴-cyclosporine A ;
[(3R,4R)-3-hydroxy-5-méthylthio-N-méthyl-L-leucine]¹[(R)-3-(1-pipéridyl)-3-méthylbutylthio-Sar]³-[4'-hydroxy-MeLeu]⁴-cyclosporine A
[(3R,4R)-3-hydroxy-5-méthylthio-N-méthyl-L-leucine]¹[(R)-2-(1-morpholino)éthylthio-Sar]³-[4'-hydroxy-MeLeu]⁴-cyclosporine A ;
[(3R,4R)-3-hydroxy-5-méthylthio-N-méthyl-L-leucine]¹[(R)-2-(1-azétidino)éthylthio-Sar]³-[4'-hydroxy-MeLeu]⁴-cyclosparine A ;
[(3R,4R)-3-hydroxy-5-méthylthio-N-méthyl-L-leucine]¹{(R)-2-[1-(4-méthylpipérazino)]éthylthio-Sar}³-[4'-hydroxy-MeLeu]⁴-cyclosporine A ;
[(3R,4R)-3-hydroxy-5-méthylthio-N-méthyl-L-leucine]¹{(R)-2-[1-(4-phénylpipérazino)]éthylthio-Sar}³-[4'-hydroxy-MeLeu]⁴-cyclosporine A ;
[(3R,4R)-3-hydroxy-5-méthylthio-N-méthyl-L-leucine]¹{(R)-2-[1-(4-benzylpipérazino)]éthylthio-Sar}³-[4'-hydroxy-MeLeu]⁴-cyclosporine A ;
[(3R,4R)-3-hydroxy-5-méthylthio-N-méthyl-L-leucine]¹{(R)-2-[1-(4-méthyl-1,2,3,6-tétrahydropyridyl)]éthylthio-Sar}³-[4'-hydroxy-MeLeu]⁴-cyclosporine A ;
[(3R,4R)-3-hydroxy-5-méthylthio-N-méthyl-L-leucine]¹{(R)-2-[1-(4-phényl-1,2,3,6-tétrahydropyridyl)]éthylthio-Sar}³-[4'-hydroxy-MeLeu]⁴-cyclosporine A ;
[(3R,4R)-3-hydroxy-5-méthylthio-N-méthyl-L-leucine]¹[(R)-3-(1-morpholino)propylthio-Sar]³-[4'-hydroxy-MeLeu]⁴-cyclosporine A ;
[(3R,4R)-3-hydroxy-5-méthylthio-N-méthyl-L-leucine]¹[(R)-3-(1-azétidino)propylthio-Sar]³-[4'-hydroxy-MeLeu]⁴-cyclosporine A ;
[(3R,4R)-3-hydroxy-5-méthylthio-N-méthyl-L-leucine]¹{(R)-3-[1-(4-méthylpipérazino)]propylthio-Sar}³-[4'-hydroxy-MeLeu]⁴-cyclosporine A ;
[(3R,4R)-3-hydroxy-5-méthylthio-N-méthyl-L-leucine]¹{(R)-3-[1-(4-phénylpipérazino)]propylthio-Sar}³-[4'-hydroxy-MeLeu]⁴-cyclosporine A ;
[(3R,4R)-3-hydroxy-5-méthylthio-N-méthyl-L-leucine]¹{(R)-3-[1-(4-benzylpipérazino)]propylthio-Sar}³-[4'-hydroxy-MeLeu]⁴-cyclosporine A ;
[(3R,4R)-3-hydroxy-5-méthylthio-N-méthyl-L-leucine]¹{(R)-3-[1-(4-méthyl-1,2,3,6-tétrahydropyridyl)]propylthio-Sar}³-[4'-hydroxy-MeLeu]⁴-cyclosporine A ;
[(3R,4R)-3-hydroxy-5-méthylthio-N-méthyl-L-leucine]¹{(R)-3-[1-(4-méthyl-1,2,3,6-tétrahydropyridyl)]propylthio-Sar}³-[4'-hydroxy-MeLeu]⁴-cyclosporine A.

La présente invention concerne également les compositions pharmaceutiques contenant au moins un produit de formule générale (I) le cas échéant sous forme de sel, à l'état pur ou sous forme d'une association avec un ou plusieurs diluants ou adjuvants compatibles et pharmaceutiquement acceptables, ou avec un autre agent antirétrovirus, éventuellement destiné au traitement du SIDA, un agent antiviral, immunomodulateur ou antimicrobien.

La composition selon l'invention est capable de maintenir en vie les cellules infectées par un rétrovirus comme par exemple le VIH et donc de réduire la progression vers le SIDA ou de diminuer sa gravité chez les sujets déjà infectés en réduisant la mortalité des cellules infectées. Les compositions peuvent être utilisées par voie orale, parentérale, rectale ou en aérosols.

Les compositions pharmaceutiques peuvent être utilisées à titre curatif ou à titre préventif chez des sujets présentant une immunodéficience et/ou infectés par un rétrovirus. Bien entendu, la constitution de ces compositions sera adaptée au cas particulier du tractus digestif des immunodéprimés.

Comme compositions solides pour administration orale peuvent être utilisés des comprimés, des pilules, des gélules, des poudres ou des granulés. Dans ces compositions, le produit actif selon l'invention est mélangé à un ou plusieurs diluants ou adjuvants inertes, tels que saccharose, lactose ou amidon.

Ces compositions peuvent comprendre des substances autres que les diluants, par exemple un lubrifiant tel que le stéarate de magnésium ou un enrobage destiné à une libération contrôlée.

Comme compositions liquides pour administration orale, on peut utiliser des solutions pharmaceutiquement acceptables, des suspensions, des émulsions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer le propylèneglycol, un polyéthylèneglycol, des huiles végétales, en particulier l'huile d'olive, des esters organiques injectables, par exemple l'oléate d'éthyle.

Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions par administration rectale sont les suppositoires ou les capsules rectales, qui contiennent outre le principe actif, des excipients tels que le beurre de cacao, des glycérides semi-synthétiques ou des polyéthylèneglycols.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine, le médecin déterminera la posologie qu'il estime la plus appropriée en fonction d'un traitement préventif ou curatif, en fonction de l'âge, du poids, du degré de l'infection et des autres facteurs propres au sujet à traiter. Généralement, les doses sont comprises entre 5 et 30 mg/kg par voie orale pour un adulte.

Il a de plus été montré que les dérivés de cyclosporine de formule générale (I) manifestent un effet de synergie lorsqu'ils sont associés à d'autres agents anti-viraux actifs sur les rétrovirus. La présente invention concerne également les associations synergisantes qui contiennent au moins un dérivé de la cyclosporine de formule générale (I) et/ou le cas échéant leurs sels, et un principe actif connu pour son activité sur les rétrovirus.

Les agents connus pour leur activité sur les rétrovirus qui peuvent être associés, sont choisis parmi des agents compatibles et inertes vis à vis du dérivé de cyclosporine de formule générale (I), aussi bien dans la catégorie des traitements pharmacologiques que dans la catégorie des traitements alternatifs tels que la thérapie génique et cellulaire ou antisens. A titre non limitatif ces agents constituant les differentes classes thérapeutiques sont choisis par exemple parmi des inhibiteurs nucléosidiques (NRTI) et non nucléosidiques (NNRTI) de la reverse transcriptase [zidovudine (AZT), didanosine (DDI), didéoxycytidine (DDC), d4T, ribavirine, 3TC, névirapine ...], parmi des inhibiteurs de la protéase [comme par exemple le Saquinavir, le Ritonavir, l'Indinavir et le Nelfinavir], des inhibiteurs de l'intégrase [comme le AR177], parmi les inhibiteurs de gene thérapie ciblant les protéines régulatrices de la réplication des VIHs tels que les inhibiteurs de la protéine rev [comme par exemple le Rev M10], ou les inhibiteurs de la nucléocapside [comme par exemple les DIBAs], parmi les inhibiteurs ciblant les transcripts RNA messager specifiques de tous les VIHs comme par exemple les anti-sens [comme GEM92, GPI-2A...], parmi les inhibiteurs de la famille des modulateurs de dNTP cellulaire (comme hydroxyurée], parmi les inhibiteurs de cytokines [comme TNF], parmi les inhibiteurs d'entrée des VIHs [comme T20, SPC-3...], ainsi que parmi les agents constituants les classes therapeutiques utilisées dans les approches vaccinales tant par biotechnologie [comme HIVAC-1e, ALVAC...] que par composés agissant sur la réponse immune [comme RG-8394].

Les compositions pharmaceutiques comprenant de telles associations, éventuellement en présence d'excipients pharmaceutiquement acceptables, entrent également dans le cadre de la présente invention.

L'exemple suivant illustre une composition selon l'invention.

### Exemple

On prépare une formulation administrable par voie orale et ayant la composition suivante :

| | |
|---|---|
| [(3R,4R)-3-hydroxy-5-méthylthio-N-méthyl-L-leucine]¹[(2R)-(2'-diéthylamino)-éthylthiosarcosine]³-cyclosporine A | 250 mg |
| Stéarate de magnésium | 3 mg |
| Acidsol | 15 mg |
| Silice colloïdale | 2 mg |
| Lactose | 130 mg |

## Revendications

1. Un dérivé de la cyclosporine **caractérisé en ce qu'**il répond à la formule générale : 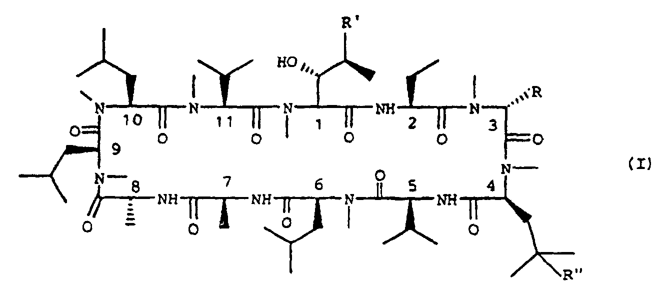 dans laquelle :
R est un atome d'hydrogène ou un radical de structure :
―S―Alk-R° (Ia)
pour lequel
- Alk-R° représente un radical méthyle, ou bien
- Alk représente un radical alcoylène contenant 2 à 6 atomes de carbone en chaîne droite ou ramifiée ou cycloalcoylène contenant 3 à 6 atomes de carbone et
- R° représente
· soit un atome d'hydrogène, un radical hydroxy, carboxy ou alcoyloxycarbonyle,
· soit un radical -NR₁R₂ pour lequel R₁ et R₂ identiques ou différents représentent des atomes d'hydrogène, ou des radicaux alcoyle, alcényle (2 à 4C) , cycloalcoyle (3 à 6C), phényle éventuellement substitué (par un atome d'halogène, alcoyloxy, alcoyloxycarbonyle, amino, alcoylamino ou dialcoylamino), ou représentent des radicaux benzyle ou hétérocyclyle saturé où insaturé contenant 5 ou 6 chaînons et 1 à 3 hétéroatomes, ou pour lequel R₁ et R₂ forment avec l'atome d'azote auquel ils sont rattachés un hétérocycle de 4 à 6 chaînons pouvant contenir un autre hétéroatome choisi parmi l'azote, l'oxygène ou le soufre et éventuellement substitué par alcoyle, phényle ou benzyle,
· soit un radical de formule générale : 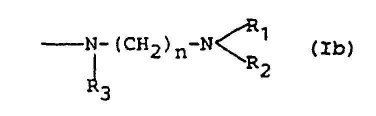
pour lequel R₁ et R₂ sont définis comme ci-dessus, R₃ représente un atome d'hydrogène ou un radical alcoyle et n est un nombre entier de 2 à 4,
R' représente un radical
-CH₂-CH=CH-CH₂-R₄ (Ic) ou - CH₂-S-R'₄ (Id)
pour lequel R₄ représente un radical alcoylthio, aminoalcoylthio, alcoylaminoalcoylthio, dialcoylaminoalcoylthio, pyrimidinylthio, thiazolylthio, N-alcoylimidazolylthio, hydroxyalcoylphénylthio, hydroxyalcolylphényloxy, nitrophénylamino, 2-oxopyrimidin-1-yle, et R'₄ représente un radical alcoyle, et
R" représente un atome d'hydrogène ou un radical hydroxy,
sous réserve que R et R" ne soient pas simultanément un atome d'hydrogène, et étant entendu que les portions ou radicaux alcoyle définis ci-dessus sont droits ou ramifiés et contiennent 1 à 4 atomes de carbone,
ainsi que ses sels pharmaceutiquement acceptables lorsqu'ils existent.

2. Un dérivé de la cyclosporine selon la revendication 1, **caractérisé en ce que**
R est un atome d'hydrogène ou un radical de structure (Ia) définie dans la revendication 1, pour lequel
- Alk-R° représente un radical méthyle, ou bien
- Alk représente un radical alcoylène contenant 2 à 6 atomes de carbone en chaîne droite ou ramifiée et R° représente un radical -NR₁R₂ pour lequel R₁ et R₂ identiques ou différents représentent des radicaux alcoyle,
R' représente un radical
-CH₂-CH=CH-CH₂-R₄ (Ic) ou - CH₂-S-R'₄ (Id)
pour lequel R₄ représente un radical alcoylthio, et R'₄ représente un radical alcoyle, et
R" représente un atome d'hydrogène ou un radical hydroxy,
sous réserve que R et R" ne soient pas simultanément un atome d'hydrogène, et étant entendu que les portions ou radicaux alcoyle définis ci-dessus sont droits ou ramifiés et contiennent 1 à 4 atomes de carbone,
ainsi que ses sels pharmaceutiquement acceptables lorsqu'ils existent.

3. Un dérivé de la cyclosporine selon la revendication 1, **caractérisé en ce que**
R est un radical de structure (Ia) définie dans la revendication 1, pour lequel
- Alk-R° représente un radical méthyle, ou bien
- Alk représente un radical éthylène et R° représente un radical -NR₁R₂ pour lequel R₁ et R₂ identiques ou différents représentent des radicaux alcoyle contenant 1 ou 2 atomes de carbone,
R' représente un radical
-CH₂-CH=CH-CH₂-R₄ (Ic) ou - CH₂-S-R'₄ (Id)
pour lequel R₄ représente un radical méthylthio, et R'₄ représente un radical méthyle, et
R" représente un atome d'hydrogène ou un radical hydroxy,
ou bien R est un atome d'hydrogène, R' est défini comme précédemment et R" est un radical hydroxy,
ainsi que leurs sels pharmaceutiquement acceptables lorsqu'ils existent.

4. Un dérivé de la cyclosporine selon la revendication 1, **caractérisé en ce qu'**il s'agit de la [(3R,4R)-3-hydroxy-5-méthylthio-N-méthyl-L-leucine]¹[(2R)-méthyl-thiosarcosine]³-cyclosporine A.

5. Un dérivé de la cyclosporine selon la revendication 1, **caractérisé en ce qu'**il s'agit de la [(3R,4R)-3-hydroxy-5-méthylthio-N-méthyl-L-leucine]¹[(2R)-(2'-diéthylamino)-éthylthiosarcosine]³-cyclosporine A, ainsi que ses sels pharmaceutiquement acceptables.

6. Un dérivé de la cyclosporine selon la revendication 1, **caractérisé en ce qu'**il s'agit de la [(3R,4R)-3-hydroxy-5-méthylthio-N-méthyl-L-leucine]¹[4-hydroxy-N-méthylleucine]⁴-cyclosporine A.

7. Un dérivé de la cyclosporine selon la revendication 1, **caractérisé en ce qu'**il s'agit de la [((4R)-4-(4-méthylthio-butèn-2-yl-(E))-N,4-diméthylthréonine]¹-[(2R)-(2'-diéthylamino)-éthylthiosarcosine]³-cyclosporine A, ainsi que ses sels pharmaceutiquement acceptables.

8. Un dérivé de la cyclosporine selon la revendication 1, **caractérisé en ce qu'**il s'agit de la [(3R,4R)-3-hydroxy-5-méthylthio-N-méthyl-L-leucine]¹ [(2R)-(2'-diméthylamino)-éthylthiosarcosine]³ [4'-hydroxy-N-méthyl-L-leucine]⁴ cyclosporine A, ainsi que ses sels pharmaceutiquement acceptables.

9. Procédé de préparation d'un dérivé de cyclosporine selon la revendication 1, **caractérisé en ce que** l'on opère :
1a) à partir de la 8'-bromo 3'-acétoxy cyclosporine de formule générale : 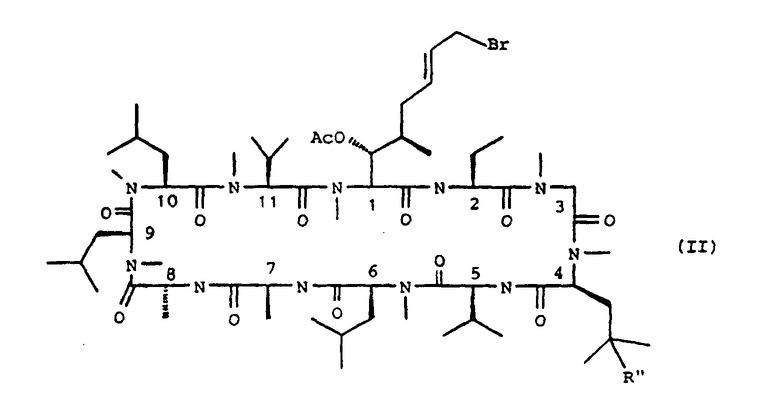 dans laquelle R" est défini comme précédemment, par action d'un thiol de formule générale :
HS-R"₄ (IIIa)
dans laquelle R"₄ représente un radical alcoyle, aminoalcoyle, alcoylaminoalcoyle, dialcoylaminoalcoyle, pyridinyle, thiazolyle, N-alcoylimidazolyle, hydroxyalcoylphényle, ou un phénol de formule générale :
HO-R'''₄ (IIIb)
dans laquelle R'''₄ représente un radical hydroxyalcoylphényle ou une amine de formule générale :
H₂N-R''''₄ (IIIc)
dans laquelle R''''₄ représente un radical nitrophényle ou de la 2-oxopyrimidine, en milieu basique, puis élimination du radical 3'-acétoxy protecteur du radical hydroxy de la chaîne en position -1,
pour obtenir un dérivé pour lequel R' est un radical de formule (Ic) tel que défini dans la revendication 1, ou bien
1b) à partir d'un anhydride mixte de l'acide de formule générale : 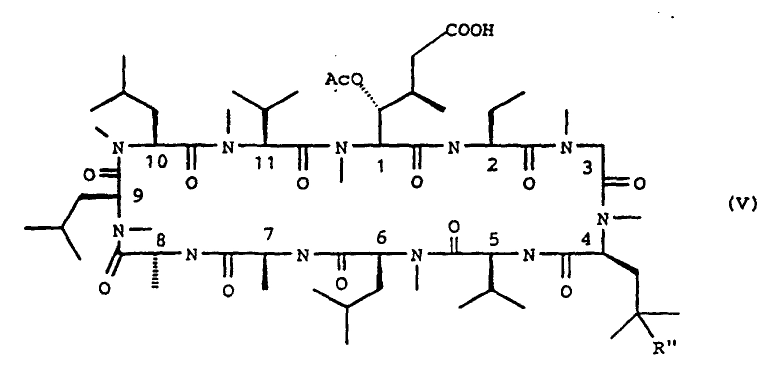 dans laquelle R" est défini comme précédemment et Ac représente un radical acétyle, tout d'abord transformé en ester thiohydroxamique par action d'un sel de la N-hydroxy-2-thiopyridine à l'abri de la lumière, puis transformé photochimiquement, par irradiation, en présence d'un disulfure de formule générale :
R'₄-S-S-R'₄ (IV)
dans laquelle R'₄ est défini comme dans la revendication 1, et débarrassé du radical acétyle protecteur d'hydroxy,
pour obtenir un dérivé pour lequel R' est un radical de formule (Id) tel que défini dans la revendication 1, puis
2) éventuellement effectue l'opération subséquente d'hydroxylation en position 4' du résidu [MeLeu]⁴ lorsque l'on veut obtenir un dérivé pour lequel R" est hydroxy et lorsque l'on a effectué la réaction au départ d'un dérivé de cyclosporine pour lequel R" est un atome d'hydrogène
3) et/ou effectue l'opération subséquente de substitution de la chaîne de formule générale (Ia) telle que définie dans la revendication 1, lorsque l'on veut obtenir un dérivé de cyclosporine pour lequel R est autre que l'atome d'hydrogène,
et transforme éventuellement le produit obtenu en un sel lorsqu'ils existent.

10. Composition pharmaceutique **caractérisée en ce qu'**elle contient au moins un produit selon la revendication 1, à l'état pur ou en association avec tout diluant ou adjuvant compatible et pharmaceutiquement acceptable et/ou en association avec un autre principe actif antiviral, immunomodulateur ou antimicrobien.

11. Associations synergisantes **caractérisées en ce qu'**elles comprennent au moins un dérivé de cyclosporine selon la revendication 1 et au moins un autre agent connu pour son activité anti-rétrovirus.

## Claims

1. Cyclosporin derivative, **characterized in that** it corresponds to the general formula: 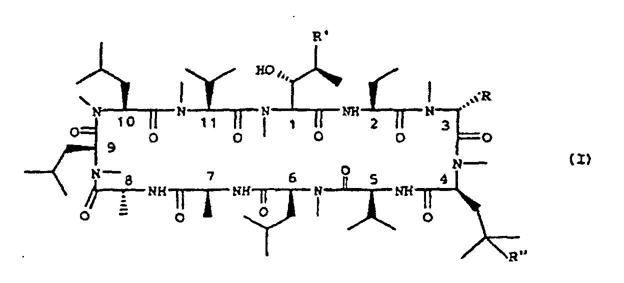 in which:
R is a hydrogen atom or a radical of structure:
―S―Alk-R^{o} (Ia)
in which
- Alk-R° represents a methyl radical, or alternatively
- Alk represents a straight- or branched-chain alkylene radical containing 2 to 6 carbon atoms or a cycloalkylene radical containing 3 to 6 carbon atoms and
- R° represents
• either a hydrogen atom or a hydroxyl, carboxyl or alkyloxycarbonyl radical,
• or an -NR₁R₂ radical in which R₁ and R₂, which are identical or different, represent hydrogen atoms or optionally substituted (by a halogen atom, alkyloxy, alkyloxycarbonyl, amino, alkylamino or dialkylamino) phenyl, alkyl, alkenyl (2 to 4C) or cycloalkyl (3 to 6C) radicals or represent benzyl or heterocyclyl radicals, the latter being saturated or unsaturated and containing 5 or 6 ring members and 1 to 3 heteroatoms, or in which R₁ and R₂ form, with the nitrogen atom to which they are attached, a 4- to 6-membered heterocycle which can contain another heteroatom chosen from nitrogen, oxygen or sulphur and which is optionally substituted by alkyl, phenyl or benzyl,
• or a radical of general formula: 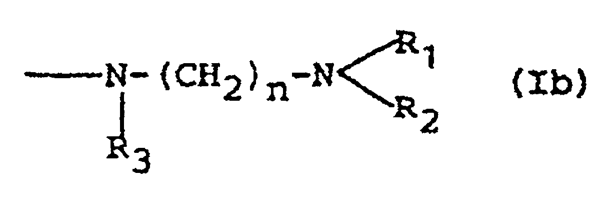
in which R₁ and R₂ are defined as above, R₃ represents a hydrogen atom or an alkyl radical and n is an integer from 2 to 4,
R' represents a radical
-CH₂-CH=CH-CH₂-R₄ (Ic) or -CH₂-S-R'₄ (Id)
in which R₄ represents an alkylthio, aminoalkylthio, alkylaminoalkylthio, dialkylaminoalkylthio, pyrimidinylthio, thiazolylthio, N-alkylimidazolylthio, hydroxyalkylphenylthio, hydroxyalkylphenyloxy, nitrophenylamino or 2-oxopyrimidin-1-yl radical and R'₄ represents an alkyl radical, and
R'' represents a hydrogen atom or a hydroxyl radical, with the proviso that R and R'' are not simultaneously a hydrogen atom and it being understood that the alkyl portions or radicals defined above are straight or branched and contain 1 to 4 carbon atoms, and its pharmaceutically acceptable salts, when they exist.

2. Cyclosporin derivative according to Claim 1, **characterized in that**
R is a hydrogen atom or a radical of structure (Ia) defined in claim 1, in which
- Alk-R° represents a methyl radical, or alternatively
- Alk represents a straight- or branched-chain alkylene radical containing 2 to 6 carbon atoms and R° represents an -NR₁R₂ radical in which R₁ and R₂, which are identical or different, represent alkyl radicals,
R' represents a radical
-CH₂-CH=CH-CH₂-R₄ (Ic) or -CH₂-S-R'₄ (Id)
in which R₄ represents an alkylthio radical and R'₄ represents an alkyl radical, and
R'' represents a hydrogen atom or a hydroxyl radical, with the proviso that R and R" are not simultaneously a hydrogen atom and it being understood that the alkyl portions or radicals defined above are straight or branched and contain 1 to 4 carbon atoms,
and its pharmaceutically acceptable salts, when they exist.

3. Cyclosporin derivative according to Claim 1, **characterized in that**
R is a radical of structure (Ia) defined in Claim 1, in which
- Alk-R° represents a methyl radical, or alternatively
- Alk represents an ethylene radical and R° represents an -NR₁R₂ radical in which R₁ and R₂, which are identical or different, represent alkyl radicals containing 1 or 2 carbon atoms,
R' represents a radical
-CH₂-CH=CH-CH₂-R₄ (Ic) or -CH₂-S-R'₄ (Id)
in which R₄ represents a methylthio radical and R'₄ represents a methyl radical, and
R'' represents a hydrogen atom or a hydroxyl radical,
or alternatively R is a hydrogen atom, R' is defined as above and R'' is a hydroxyl radical,
and its pharmaceutically acceptable salts, when they exist.

4. Cyclosporin derivative according to Claim 1, **characterized in that** it is [(3R,4R)-3-hydroxy-5-methylthio-N-methyl-L-leucine]¹[(2R)-methylthiosarcosine]³-cyclosporin A.

5. Cyclosporin derivative according to Claim 1, **characterized in that** it is [(3R,4R)-3-hydroxy-5-methylthio-N-methyl-L-leucine]¹[(2R)-2'-(diethylamino)-ethylthiosarcosine]³-cyclosporin A, and its pharmaceutically acceptable salts.

6. Cyclosporin derivative according to Claim 1, **characterized in that** it is [(3R,4R)-3-hydroxy-5-methylthio-N-methyl-L-leucine]¹[4-hydroxy-N-methyl-leucine]⁴-cyclosporin A.

7. Cyclosporin derivative according to Claim 1, **characterized in that** it is [(4R)-4-((E)-4-(methylthio)buten-2-yl)-N,4-dimethylthreonine]¹-[(2R)-2'-(diethylamino)ethylthiosarcosine]³-cyclosporin A, and its pharmaceutically acceptable salts.

8. Cyclosporin derivative according to Claim 1, **characterized in that** it is [(3R,4R)-3-hydroxy-5-methylthio-N-methyl-L-leucine]¹[(2R)-2'-(dimethylamino)ethylthiosarcosine]³[4'-hydroxy-N-methyl-L-leucine]⁴-cyclosporin A, and its pharmaceutically acceptable salts.

9. Process for the preparation of a cyclosporin derivative according to Claim 1, **characterized in that** the preparation is carried out:
1a) from the 8'-bromo-3-acetoxycyclosporin of general formula: 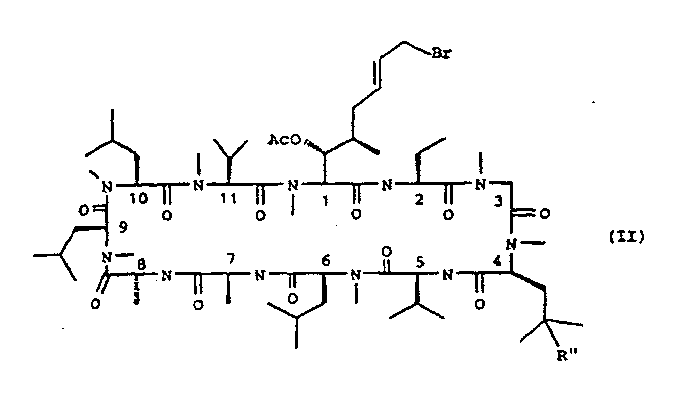 in which R'' is defined as above, by reaction with a thiol of general formula:
HS-R''₄ (IIIa)
in which R''₄ represents an alkyl, aminoalkyl, alkylaminoalkyl, dialkylaminoalkyl, pyridinyl, thiazolyl, N-alkylimidazolyl or hydroxyalkylphenyl radical, or a phenol of general formula:
HO-R'''₄ (IIIb)
in which R'''₄ represents a hydroxyalkylphenyl radical, or an amine of general formula:
H₂N-R''''₄ (IIIc)
in which R''''₄ represents a nitrophenyl radical or a radical of 2-oxopyrimidine, in basic medium, and then removal of the 3'-acetoxy protective radical of the hydroxyl radical of the chain at the 1-position, in order to obtain a derivative in which R' is a radical of formula (Ic) as defined in Claim 1, or alternatively
Ib) from a mixed anhydride of the acid of general formula: 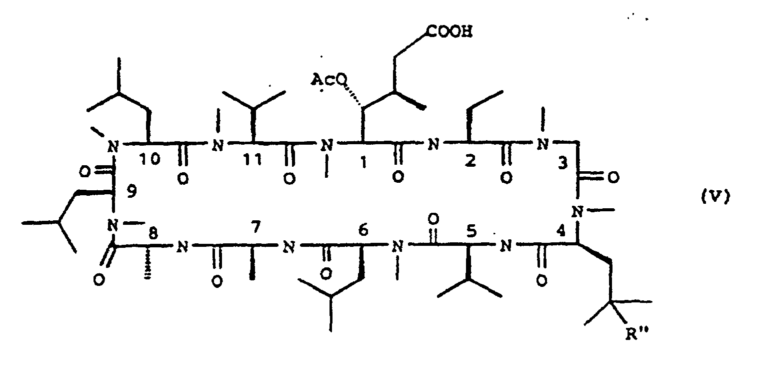 in which R" is defined as above and Ac represents an acetyl radical, first of all converted to the thiohydroxamic ester by reaction with a salt of N-hydroxy-2-thiopyridine with the exclusion of light, then photochemically converted, by irradiation, in the presence of a disulphide of general formula:
R'₄-S-S-R'₄ (IV)
in which R'₄ is defined as in Claim 1, and freed from the hydroxy-protective acetyl radical,
in order to obtain a derivative in which R' is a radical of formula (Id) as defined in Claim 1, then
2) the subsequent operation of hydroxylation at the 4'-position of the [MeLeu]⁴ residue is optionally carried out when it is desired to obtain a derivative in which R'' is hydroxyl and when the reaction has been carried out starting from a cyclosporin derivative in which R'' is a hydrogen atom
3) and/or the subsequent operation of substitution of the chain of general formula (Ia) as defined in Claim 1 is carried out when it is desired to obtain a cyclosporin derivative in which R is other than the hydrogen atom, and the product obtained is optionally converted into a salt, when they exist.

10. Pharmaceutical composition, **characterized in that** it contains at least one product according to Claim 1, in the pure state or in combination with any compatible and pharmaceutically acceptable diluent or adjuvant and/or in combination with another antiviral, immunomodulating or antimicrobial active principle.

11. Synergizing combinations, **characterized in that** they comprise at least one cyclosporin derivative according to Claim 1 and at least one other agent known for its anti-retrovirus activity.

## Patentansprüche

1. Cyclosporin-Derivat, **dadurch gekennzeichnet, daß** es der allgemeinen Formel (I) entspricht: 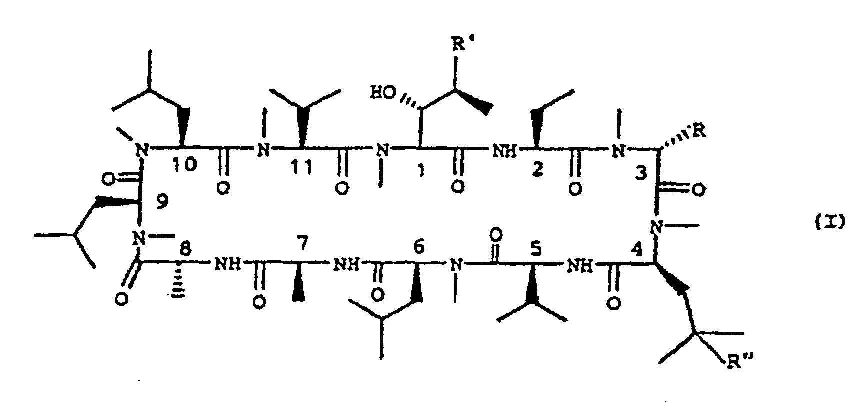 in der
R ein Wasserstoffatom oder ein Rest der Struktur (Ia)
-S-Alk-R° (Ia)
ist, worin
- Alk-R° einen Rest Methyl darstellt, oder auch
- Alk einen Rest Alkylen mit 2 bis 6 Kohlenstoffatomen in
gerader oder verzweigter Kette oder Cycloalkylen mit 3 bis 6 Kohlenstoffatomen darstellt und
- R° darstellt:
· entweder ein Wasserstoffatom, einen Rest Hydroxy, Carboxy oder Alkyloxycarbonyl,
· oder einen Rest -NR₁R₂, worin R₁ und R₂, gleich oder verschieden, Wasserstoffatome oder Reste Alkyl, Alkenyl (2 bis 4 Kohlenstoffe), Cycloalkyl (3 bis 6 Kohlenstoffe), Phenyl, gegebenenfalls substituiert (durch ein Halogenatom, Alkyloxy, Alkyloxycarbonyl, Amino, Alkylamino oder Dialkylamino) darstellen, oder Reste Benzyl oder Heterocyclyl, gesättigt oder ungesättigt und mit 5 oder 6 Kettengliedern und 1 bis 3 Heteroatomen, bedeuten, oder worin R₁ und R₂ zusammen mit dem Stickstoffatom, an das sie gebunden sind, einen Heterocyclus mit 4 bis 6 Kettengliedern bilden, der ein anderes Heteroatom enthalten kann, ausgewählt unter Stickstoff, Sauerstoff oder Schwefel und gegebenenfalls substituiert ist durch Alkyl, Phenyl oder Benzyl,
· oder einen Rest der allgemeinen Formel (Ib): 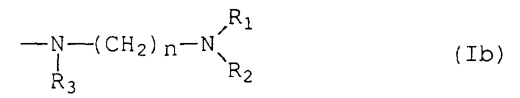
· worin R₁ und R₂ wie oben definiert sind, R₃ ein Wasserstoffatom oder einen Rest Alkyl darstellt und n eine ganze Zahl von 2 bis 4 ist,
R' einen Rest
―CH₂―CH=CH-CH₂―R₄ (Ic) oder ―CH₂-S―R'₄ (Id)
bedeutet,
worin R₄ einen Rest Alkylthio, Aminoalkylthio, Alkylaminoalkylthio, Dialkylaminoalkylthio, Pyrimidinylthio, Thiazolylthio, N-Alkylimidazolylthio, Hydroxyalkylphenylthio, Hydroxyalkylphenyloxy, Nitrophenylamino, 2-Oxopyrimidin-1-yl darstellt und
R'₄ ein Rest Alkyl ist, und
R" ein Wasserstoffatom oder einen Rest Hydroxy bedeutet,
unter dem Vorbehalt, daß R und R" nicht gleichzeitig ein Wasserstoffatom sind und mit der Maßgabe, daß die wie oben definierten Teile oder Reste Alkyl gerade oder verzweigt sind und 1 bis 4 Kohlenstoffatome enthalten, sowie ihre pharmazeutisch akzeptablen Salze, wenn sie existieren.

2. Cyclosporin-Derivat nach Anspruch 1, **dadurch gekennzeichnet, daß**
R ein Wasserstoffatom oder ein Rest der wie in Anspruch 1 definierten Struktur (Ia) ist, worin
- Alk-R° einen Rest Methyl darstellt, oder auch
- Alk einen Rest Alkylen mit 2 bis 6 Kohlenstoffatomen in
gerader oder verzweigter Kette darstellt und R° einen Rest
-NR₁R₂ darstellt, worin R₁ und R₂, gleich oder verschieden,
Reste Alkyl bedeuten,
R' einen Rest
―CH₂―CH=CH-CH₂―R₄ (Ic) oder ―CH₂-S―R'₄ (Id)
bedeutet,
worin R₄ einen Rest Alkylthio darstellt und R'₄ ein Rest Alkyl ist, und
R" ein Wasserstoffatom oder einen Rest Hydroxy bedeutet,
unter dem Vorbehalt, daß R und R" nicht gleichzeitig ein Wasserstoffatom sind und mit der Maßgabe, daß die wie oben definierten Teile oder Reste Alkyl gerade oder verzweigt sind und 1 bis 4 Kohlenstoffatome enthalten,
sowie ihre pharmazeutisch akzeptablen Salze, wenn sie existieren.

3. Cyclosporin-Derivat nach Anspruch 1, **dadurch gekennzeichnet, daß**
R ein Rest der wie in Anspruch 1 definierten Struktur (Ia) ist, worin
- Alk-R° einen Rest Methyl darstellt, oder auch
- Alk einen Rest Ethylen darstellt und R° einen Rest -NR₁R₂ bedeutet, worin R₁ und R₂, gleich oder verschieden,
Reste Alkyl mit 1 oder 2 Kohlenstoffatomen darstellen,
R' einen Rest
―CH₂―CH=CH―CH₂―R₄ (Ic) oder ―CH₂-S―R'₄ (Id)
bedeutet,
worin R₄ einen Rest Methylthio darstellt und R'₄ ein Rest Methyl ist, und
R" ein Wasserstoffatom oder einen Rest Hydroxy bedeutet,
oder auch R ein Wasserstoffatom darstellt, R' wie vorstehend definiert ist und R" ein Rest Hydroxy ist,
sowie ihre pharmazeutisch akzeptablen Salze, wenn sie existieren.

4. Cyclosporin-Derivat nach Anspruch 1, **dadurch gekennzeichnet, daß** es sich um das [(3R,4R)-3-Hydroxy-5-methylthio-N-methyl-L-leucin]¹[(2R)-methylthiosarcosin]³-cyclosporin A handelt.

5. Cyclosporin-Derivat nach Anspruch 1, **dadurch gekennzeichnet, daß** es sich um das [(3R,4R)-3-Hydroxy-5-methylthio-N-methyl-L-leucin]¹[(2R)-(2'-diethylamino)-ethyl-thiosarcosin]³-cyclosporin A handelt, sowie seine pharmazeutisch akzeptablen Salze.

6. Cyclosporin-Derivat nach Anspruch 1, **dadurch gekennzeichnet, daß** es sich um das [(3R,4R)-3-Hydroxy-5-methylthio-N-methyl-L-leucin]¹[4-hydroxy-N-methylleucin]⁴-cyclosporin A handelt.

7. Cyclosporin-Derivat nach Anspruch 1, **dadurch gekennzeichnet, daß** es sich um das [((4R)-4-(4-Methylthio-buten-2-yl-(E))-N,4-dimethylthreonin]¹[(2R)-(2'-diethylamino)-ethyl-thiosarcosin]³-cyclosporin A handelt, sowie seine pharmazeutisch akzeptablen Salze.

8. Cyclosporin-Derivat nach Anspruch 1, **dadurch gekennzeichnet, daß** es sich um das [(3R,4R)-3-Hydroxy-5-methylthio-N-methyl-L-leucin]¹[(2R)-(2'-dimethylamino)-ethyl-thiosarcosin]³-[4'-hydroxy-N-methyl-L-leucin]⁴cyclosporin A handelt, sowie seine pharmazeutisch akzeptablen Salze.

9. Verfahren zur Herstellung eines Cyclosporin-Derivates nach Anspruch 1, **dadurch gekennzeichnet, daß** man verfährt:
1a) ausgehend von 8'-Brom-3'-acetoxy-cyclosporin der allgemeinen Formel (II) 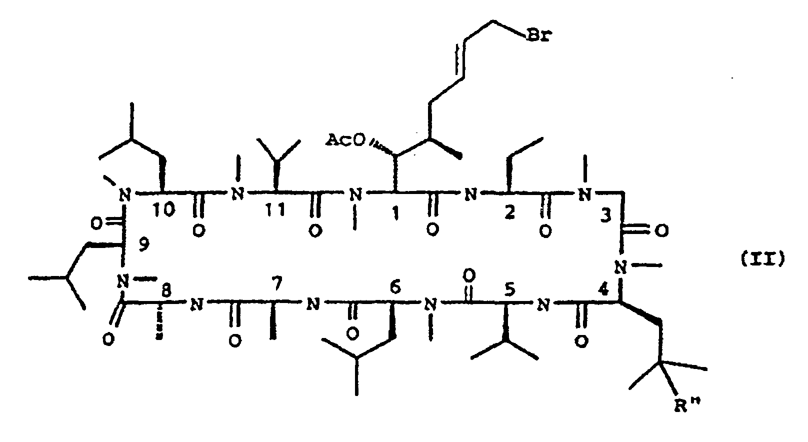 in der R" wie vorstehend definiert ist, durch Einwirkung eines Thiols der allgemeinen Formel (IIIa)
HS-R"₄ (IIIa)
in der
R"₄ einen Rest Alkyl, Aminoalkyl, Alkylaminoalkyl, Dialkylaminoalkyl, Pyridinyl, Thiazolyl, N-Alkylimidazolyl, Hydroxyalkylphenyl darstellt, oder eines Phenols der allgemeinen Formel (IIIb)
HO-R"'₄ (IIIb)
in der
R"'₄ einen Rest Hydroxyalkylphenyl darstellt, oder eines Amins der allgemeinen Formel (IIIc)
H₂N-R""₄ (IIIc)
in der
R""₄ einen Rest Nitrophenyl darstellt, oder von 2-Oxopyrimidin, im basischen Medium und anschließende Entfernung des Restes 3'-Acetoxy als Schutzgruppe des Restes Hydroxy der Kette in Position -1,
um ein Derivat zu erhalten, in dem R' ein wie in Anspruch 1 definierter Rest der Formel (Ic) ist, oder auch
1b) ausgehend von einem gemischten Anhydrid der Säure der allgemeinen Formel (V) 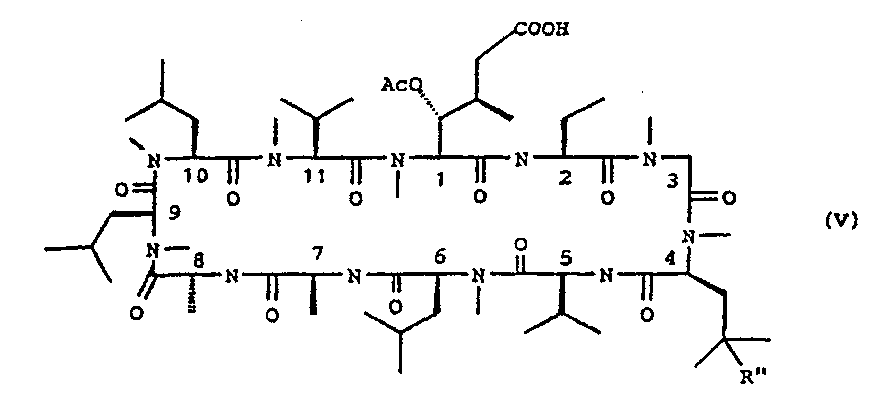 in der R" wie vorstehend definiert ist und Ac einen Rest Acetyl bedeutet, zuerst Umwandlung in den Thiohydroxamin-Ester durch Einwirkung eines Salzes von N-Hydroxy-2-thiopyridin unter Ausschluß von Licht und anschließende photochemische Umwandlung durch Strahlung in Anwesenheit eines Disulfides der allgemeinen Formel (IV)
R'₄-S-S-R'₄ (IV)
in der
R'₄ wie in Anspruch 1 definiert ist, und Entfernung des Restes Acetyl als Schutzgruppe des Restes Hydroxy, um ein Derivat zu erhalten, in dem R' ein wie in Anspruch 1 definierter Rest der Formel (Id) ist, und anschließend
2) gegebenenfalls Durchführung einer nachfolgenden Operation der Hydroxylierung in Position 4' des Restes [MeLeu]⁴, wenn man wünscht, ein Derivat zu erhalten, in dem R" Hydroxy ist und wenn man die Reaktion ausgehend von einem Cyclosporin-Derivat durchgeführt hat, in dem R" ein Wasserstoffatom ist,
3) und/oder Durchführung einer nachfolgenden Operation der Substitution der wie in Anspruch 1 definierten Kette der allgemeinen Formel (Ia), wenn man wünscht, ein Cyclosporin-Derivat zu erhalten, in dem R anders ist als das Wasserstoffatom,
und gegebenenfalls Umwandlung des erhaltenen Produktes in ein Salz, wenn sie existieren.

10. Pharmazeutische Zusammensetzung, **dadurch gekennzeichnet, daß** sie mindestens ein Produkt nach Anspruch 1 in reinem Zustand oder in Assoziation mit jedem kompatiblen und pharmazeutisch akzeptablen Verdünnungsmittel oder Zusatzstoff und/oder in Assoziation mit einem anderen antiviralen, immunomodulierenden oder antimikrobiellen Wirkstoff enthält.

11. Synergistische Assoziationen, **dadurch gekennzeichnet, daß** sie mindestens ein Cyclosporin-Derivat nach Anspruch 1 und mindestens ein anderes Mittel umfassen, das für seine Anti-Retrovirus-Aktivität bekannt ist.
